# EUROPEAN PATENT SPECIFICATION

(11) **EP 2 234 674 B1**
(45) Date of publication and mention of the grant of the patent: **29.03.2017**
(21) Application number: 08861181.9
(22) Date of filing: 12.11.2008
(51) Int. Cl.: A61Q 17/04, A61K 8/06, A61K 8/81, A61K 8/86

(54) **FLUID OIL-IN-WATER SUNSCREEN EMULSIONS CONTAINING A SELECTED GEMINI SURFACTANT AND A CROSSLINKED COPOLYMER OF METHACRYLIC ACID AND OF ETHYL ACRYLATE**
DÜNNFLÜSSIGE OEL IN WASSER SONNENSCHUTZEMULSIONEN ENTHALTEND EIN AUSGEWÄHLTES DIMERTENSID UND EIN VERNETZTES COPOLYMER VON METHACRYLSÄURE UND ETHYLACRYLAT
ÉMULSIONS ANTISOLAIRES FLUIDES HUILE DANS EAU CONTENANT UN TENSIOACTIF JUMELÉ SPECIFIQUE ET UN COPOLYMÈRE RÉTICULÉ D'ACIDE MÉTHACRYLIQUE ET D'ACRYLATE D'ÉTHYLE

(30) Priority: 18.12.2007 FR 0759955; 27.12.2007 US 16904
(43) Date of publication of application: 06.10.2010
(73) Proprietor: L'Oréal, 75008 Paris (FR)
(72) Inventor: CANDAU, Didier, F-91570 Bievres (FR); BOUTELET, Karl, F-75011 Paris (FR)
(74) Representative: Miszputen, Laurent
(86) International application number: PCT/EP2008/065393
(87) International publication number: WO 2009/077272

(56) References cited:
- WO-A-2004/105704
- US-A1- 2005 031 653
- US-A1- 2005 063 925
- ANONYMOUS: "ALLIANZ OPT Oil Phase Technology to make sunscreens water-resistant" INTERNET, [Online] XP002493228 online Retrieved from the Internet: URL:http://www.ispcorp.com/isp/products/ha irskin/content/skincare/brochure/allianzOP T/AllianzOPT.pdf> [retrieved on 2008-08-21]
- KWETKAT K: "Gemini surfactant blends for personal care applications" SOFW-JOURNAL SEIFEN, OELE, FETTE, WACHSE, VERLAG FUR CHEMISCHE INDUSTRIE, AUGSBURG, DE, vol. 128, no. 4, 1 January 2002 (2002-01-01), pages 38,40-42,44, XP001536316 ISSN: 0942-7694
- KWETKAT ET AL: "FORMULATION OF HOMOGENEOUS O/W EMULSION PUMP FOAMS" SPECIALITY CHEMICALS, REDHILL, GB, vol. 25, no. 9, 1 January 2005 (2005-01-01), pages 38-39, XP009083493 ISSN: 0262-2262

## Description

The present invention relates to a photoprotective composition containing, in a cosmetically acceptable medium,
a) as liquid phase, an oil-in-water emulsion, emulsified with at least one dimeric surfactant comprising two surfactant units, which may be identical or different, each being constituted of a hydrophilic head and a hydrophobic tail and connected to one another, via the hydrophilic heads, by means of a spacer group,
b) at least one organic UV screen and/or at least one mineral UV screen, and
c) at least one crosslinked copolymer of methacrylic acid and of ethylacrylate.

The present invention therefore also relates to a process for preparing said composition.

It is known that light radiation with wavelengths of between 280 nm and 400 nm enables browning of the human epidermis, and that rays with wavelengths of more particularly between 280 and 320 nm, known as UV-B radiation, cause erythemas and skin burns which may be harmful to the development of natural tanning. For these reasons, and also for aesthetic reasons, there is a constant demand for means of controlling this natural tanning with a view to thus controlling the colour of the skin; it is therefore advisable to screen out this UV-B radiation.

It is also known that UV-A rays, with wavelengths between 320 and 400 nm, which cause browning of the skin, are capable of inducing a detrimental change in the latter, in particular in the case of sensitive skin or of skin continually exposed to solar radiation. UV-A rays cause in particular a loss of elasticity of the skin and the appearance of wrinkles, resulting in premature aging of the skin. They promote triggering of the erythemal reaction or accentuate this reaction in certain individuals and may even be the cause of phototoxic or photoallergic reactions. Thus, for aesthetic and cosmetic reasons such as conservation of the natural elasticity of the skin, for example, more and more people wish to control the effect of UV-A rays on their skin. It is therefore desirable to also filter out UV-A radiation.

Antisun compositions comprising organic screens and/or mineral screens that are active in the UV-A range and/or active in the UV-B range are generally used for the purpose of ensuring protection of the skin and of the keratin materials against UV radiation.

Many cosmetic compositions for photoprotection of the skin have been proposed to date. These compositions generally contain, in an emulsified liquid carrier (preferably oil-in-water emulsion), one or more organic molecules capable of absorbing ultraviolet radiation, which are soluble in the oily and/or aqueous phase. The use of mineral pigments of a metal oxide, such as titanium dioxide, in such antisun compositions is increasingly common since these particles, which are invisible to the naked eye due to their small size, make it possible to increase the protection factor of the compositions containing them.

Among these known antisun products, fluid oil-in-water emulsions are, in general, more appreciated by consumers than thicker emulsions since they have a more pleasant feel and allow the product to be applied more easily.

One of the major drawbacks of these fluid antisun emulsions containing organic screens and/or mineral screens lies in the difficulty in reconciling good stability of the product and effective protection. In fact, to obtain emulsions of low viscosity, it is known practice to use extremely shearing stirring means such as high-pressure homogenizers. The prduction of emulsions with such equipment effectively makes it possible to obtain very fluid O/W dispersions, but it is expensive and especially makes it extremely difficult to introduce UV screens in the solid state, in particular mineral screens, during production. In fact, said screens are not compatible with this equipment, either because the size of the particles can clog the orifices of this equipment, or because the abrasive or even destructive nature of very hard mineral screens, such as titanium dioxide, introduces high pressures into this equipment.

One solution consists in using surfactants which, by virtue of their physicochemical characteristics, make it possible to prepare emulsions which are intrinsically fluid, i.e. without the use of very powerful dispersing tools. This is in particular the case of "gemini" surfactants, which will be defined below and which make it possible to obtain fluid compostions.

Such gemini surfactants have been described in applications DE19943681, DE19943668, WO 03024412, WO9740124; WO9731890; DE19750246; DE 19750245; DE 19631225; DE 19647060.

The stability of such emulsions is in general limited over time, the viscosity of the antisun product being insufficient to maintain the dispersed phase, constituted of oil and organic UV screens and/or mineral screens, in suspension over a period of time which is compatible with the marketing and the use of the products by the consumer. The product thus no longer has the organoleptic amd effectiveness qualities sufficient for marketing thereof.

In order to obtain fluid O/W antisun emulsions that are sufficiently stable, it is common practice to use agents for thickening/gelling the dispersant phase of the emulsion in order to maintain the dispersed phase in suspension. This is in particular the case of thickeners such as xanthan gum in the examples of application WO03/024412 or else associative polymers such as those proposed in application EP1502582. These thickeners/gelling agents need, during the production of emulsions, to be incorporated into the water and mixed for a sufficiently long period of time to be completely homogenized in the aqueous phase. Furthermore, the drawback of such a method is that, the more one seeks to stabilize the fluid emulsion by gelling the continuous phase, the more one loses the fluidity which is the desired characteristic of the product.

There thus remains a need to prepare fluid oil-in-water photoprotective emulsions containing organic UV screens and/or mineral screens, which, by simple mixing of the various components, i.e. of the emulsified liquid phase, are stable over time and easy and pleasant to apply while at the same time providing effective antisun protection.

The Applicant has noted, surprisingly, that the combined use of at least one gemini surfactant and of at least one crosslinked copolymer of methacrylic acid and of ethylacrylate makes it possible to prepare emulsified fluid antisun compositions containing organic and/or mineral UV screens without having recourse to high-pressure homogenization techniques.

These discoveries form the basis of the present invention.

According to the invention, the term "fluid" compositions is intended to mean compositions having a viscosity of, for example, less than or equal to 200 mPa.s, and preferably of from 10 to 180 mPa.s. The viscosity is measured at 25°C with a measuring apparatus of Rheomat RM180 type at a shear rate gradient of 200 s⁻¹. This apparatus is equipped with a different rotor according to the viscosities; in the case of the invention, a rotor 2 for viscosity ranges less than 200 mPa.s.

The surfactants which contribute to solving the problem of the present invention are dimeric surfactants, most commonly referred to as gemini surfactants, comprising two surfactant units, each being constituted of a hydrophilic head and a hydrophobic tail, and connected to one another, via the hydrophilic heads, by means of a spacer group.

The term "cosmetically acceptable" is intended to mean compatible with the skin and/or its appendages, which has a pleasant colour, odour and feel and which does not cause any unacceptable discomfort (stinging, tautness, redness), capable of dissuading the consumer from using this composition.

A subject of the present invention is therefore a photoprotective composition containing, in a cosmetically acceptable medium,
a) as liquid phase, an oil-in-water emulsion, emulsified with at least one specific dimeric surfactant comprising two surfactant units, which may be identical or different, each being constituted of a hydrophilic head and a hydrophobic tail and connected to one another, via the hydrophilic heads, by means of a spacer group,
b) at least one organic UV screen and/or at least one mineral UV screen, and
c) at least one crosslinked copolymer of methacrylic acid and of ethylacrylate.

Other characteristics, aspects and advantages of the invention will become apparent on reading the detailed description which follows.

### Gemini surfactants

The dimeric surfactants or gemini surfactants used in the present invention are known. For a detailed description of the various chemical structures and of the physicochemical properties thereof, reference may be made to the following publications:
Milton J. Rosen, Gemini Surfactants, Properties of surfactant molecules with two hydrophilic groups and two hydrophobic groups, Cosmetics & Toiletries magazine, vol. 113, December 1998, pages 49 - 55,
Milton J. Rosen, Recent Developments in Gemini Surfactants, Allured's Cosmetics & Toiletries magazine, July 2001, vol 116, no. 7, pages 67 - 70.

The gemini surfactants that are used in the present invention are some of those given in German Patent Application DE 199 43 681 A1, i.e.:
(i) the compounds of formula (I), described in WO96/14926 : 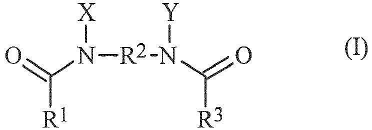 in which
   R¹ and R³ represent a linear or branched C₅-C₂₅ alkyl group, which is saturated or contains up to two non-vicinal unsaturations,
   R² represents a C₁-C₁₂ alkylene group, X and Y each represent a (C₂H₄O)ₓ(C₃H₆O)_{y}-RF group with x = 0 - 15,
   y = 0-10, x + y ≥ 1, and RF = -SO₃M, -CH₂-CO₂M, -P(O)(OM)₂, H, -C₃H₆SO₃M, or a -CH₂(CHOH)₄CH₂OH group when x + y = 0, and
   M represents an alkali metal ion, (alkyl)ammonium, alkanolammonium, H or a 1/2 alkaline-earth metal ion.

In this family of surfactants, preference is in particular given to those in which R¹ and R³ are identical and each represent a linear C₈-C₁₆ alkyl group, R² represents a C₂-C₈ alkylene group, X and Y each represent a -(C₂H₄O)x-RF group with x = 10-15 and RF = -SO₃M, where M is an alkali metal atom. A preferred gemini surfactant of this family is an anionic compound Sodium Dicocoylethylenediamine PEG-15 Sulphate (INCI name) of formula:

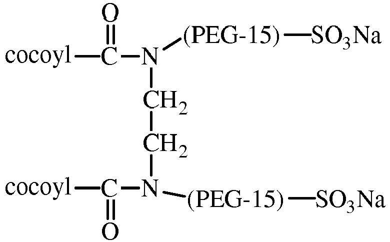

This gemini surfactant may, for example, be used in the following mixtures sold by the company Sasol under the name Ceralution®:
- Ceralution® H: Behenyl Alcohol, Glyceryl Stearate, Glyceryl Stearate Citrate and Sodium Dicocoylethylenediamine PEG-15 Sulphate,
- Ceralution® F: Sodium Lauroyl Lactylate and Sodium Dicocoylethylenediamine PEG-15 Sulphate.
- Ceralution® C: Aqua, Capric/Caprylic triglyceride, Glycerin, Ceteareth-25, Sodium Dicocoylethylenediamine PEG-15 Sulphate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben and Isobutylparaben (INCI names).

The mixture of Behenyl Alcohol, Glyceryl Stearate, Glyceryl Stearate Citrate and Sodium Dicocoylethylenediamine PEG-15 Sulphate (Ceralution® H) will more particularly be used.

The concentration of the gemini surfactant(s) used in the present invention preferably ranges from 0.001% to 8%, preferably from 0.01% to 4%, and in particular from 0.05% to 3%, relative to the total weight of the photoprotective composition.

### Crosslinked copolymer of methacrylic acid and ethyl acrylate

One of the essential characteristics of the invention is the presence of a crosslinked copolymer of methacrylic acid and of ethyl acrylate.

The methacrylic acid is preferably present in amounts ranging from 20% to 80% by weight, and more particularly from 25% to 70% by weight, and even more particularly from 35% to 65% by weight, relative to the total weight of the copolymer.

The ethyl acrylate is preferably present in amounts ranging from 15% to 80% by weight, and more particularly from 25% to 75% by weight, and even more particularly from 35% to 65% by weight, relative to the total weight of the copolymer.

This copolymer is preferably partially or completely crosslinked with at least one conventional ethylenically polyunsaturated crosslinking agent, such as polyalkenyl ethers of sucrose or of polyols, diallyl phthalates, divinylbenzene, allyl (meth)acrylate, ethylene glycol di(meth)acrylate, methylenebisacrylamide, trimethylolpropane tri(meth)acrylate, diallyl itaconate, diallyl fumarate, diallyl maleate, zinc (meth)acrylate, or derivatives of castor oil or of polyols produced from unsaturated carboxylic acids. The content of crosslinking agent ranges, in general, from 0.01% to 5% by weight, and preferably from 0.03% to 3% by weight, and even more particularly from 0.05% to 1% by weight, relative to the total weight of the copolymer.

According to one particularly preferred embodiment, the copolymer of the invention may be in particular in the form of a dispersion in water. The average size of the copolymer particles in the dispersion is generally between 10 and 500 nm, and preferably between 20 and 200 nm, and more preferentially from 50 to 150 nm.

Mention may in particular be made of the crosslinked methacrylic acid/ethyl acrylate copolymer sold by the company Noveon under the trade name Carbopol Aqua SF1.

The concentration of copolymer preferably ranges from 0.01% to 5% by weight with respect to active material, relative to the total weight of the composition, and preferably from 0.01% to 2% by weight with respect to active material, relative to the total weight of the composition.

### Agents for screening out UV radiation

The compositions in accordance with the invention comprise at least one organic or inorganic UV screen which is active in the UVA range and/or in the UVB range, and which is water-soluble or liposoluble or else insoluble in the cosmetic solvents commonly used.

Of course, those skilled in the art will take care to select the possible screen(s) and/or amounts thereof in such a way that the advantageous properties intrinsically associated with the compositions in accordance with the invention are not, or are not substantially, impaired by the additions envisaged, in particular the improvement of the stability of the emulsion.

### (i) Organic UV screens

The organic screens are in particular chosen from anthranilates; cinnamic derivatives; dibenzoylmethane derivatives; salicylic derivatives, camphor derivatives; benzophenone derivatives; β,β-diphenylacrylate derivatives; triazine derivatives; benzotriazole derivatives; benzalmalonate derivatives, in particular those mentioned in patent US 5 624 663; benzimidazole derivatives; imidazolines; the bisbenzoazolyl derivatives as described in patents EP669323 and US 2,463,264; p-aminobenzoic acid (PABA) derivatives; the methylenebis(hydroxyphenylbenzotriazole) derivatives as described in applications US 5,237,071, US 5,166,355, GB 2303549, DE 197 26 184 and EP 893119; the benzoxazole derivatives as described in patent applications EP0832642, EP1027883, EP1300137 and DE10162844; screening polymers and screening silicones such as those described in particular in application WO-93/04665; α-alkylstyrene-derived dimers such as those described in patent application DE19855649; the 4,4-diarylbutadienes as described in applications EP0967200, DE19746654, DE19755649, EP-A-1008586, EP1133980 and EP133981; merocyanin derivatives such as those described in applications WO 04/006878, WO 05/058269 and WO 06/032741, and mixtures thereof.

As examples of complementary organic photoprotective agents, mention may be made of those denoted below under their INCI name:

### Cinnamic derivatives :

Ethylhexyl Methoxycinnamate sold in particular under the trade name Parsol MCX by DSM Nutritional Products Inc.,
Isopropyl Methoxycinnamate, Isoamyl Methoxycinnamate sold under the trade name Neo Heliopan E 1000 by Symrise,
DEA Methoxycinnamate,
Diisopropyl Methylcinnamate,
Glyceryl Ethylhexanoate Dimethoxycinnamate.

### Dibenzoylmethane derivatives :

Butyl Methoxydibenzoylmethane sold in particular under the trade name Parsol 1789 by DSM Nutritional Products Inc.,
Isopropyldibenzoylmethane.

### para-Aminobenzoic acid derivatives :

PABA,
Ethyl PABA,
Ethyl Dihydroxypropyl PABA,
Ethylhexyl dimethyl PABA sold in particular under the name "Escalol 507" by ISP,
Glyceryl PABA,
PEG-25 PABA sold under the name "Uvinul P25" by BASF.

### Salicylic derivatives :

Homosalate sold under the name "Eusolex HMS" by Rona/EM Industries,
Ethylhexyl Salicylate sold under the name "Neo Heliopan OS" by Symrise,
Dipropyleneglycol Salicylate sold under the name "Dipsal" by Scher,
TEA Salicylate, sold under the name "Neo Heliopan TS" by Symrise.

### β,β-diphenylacrylate derivatives :

Octocrylene sold in particular under the trade name "Uvinul N539" by BASF,
Etocrylene sold in particular under the trade name "Uvinul N35" by BASF.

### Benzophenone derivatives :

Benzophenone-1 sold under the trade name "Uvinul 400" by BASF,
Benzophenone-2 sold under the trade name "Uvinul D50" by BASF,
Benzophenone-3 or Oxybenzone sold under the trade name "Uvinul M40" by BASF,
Benzophenone-4 sold under the trade name "Uvinul MS40" by BASF,
Benzophenone-5,
Benzophenone-6 sold under the trade name "Helisorb 11" by Norquay,
Benzophenone-8 sold under the trade name "Spectra-Sorb UV-24" by American Cyanamid,
Benzophenone-9 sold under the trade name "Uvinul DS-49" by BASF,
Benzophenone-12,
n-hexyl 2-(4-diethylamino-2-hydroxybenzoyl)benzoate sold under the trade name "Uvinul A +" by BASF.

### Benzylidenecamphor derivatives :

3-Benzylidenecamphor manufactured under the name "Mexoryl SD" by Chimex,
4-Methylbenzylidenecamphor sold under the name "Eusolex 6300" by Merck,
Benzylidenecamphorsulphonic Acid manufactured under the name "Mexoryl SL" by Chimex,
Camphor Benzalkonium Methosulphate manufactured under the name "Mexoryl SO" by Chimex,
Terephthalylidenedicamphorsulphonic acid manufactured under the name "Mexoryl SX" by Chimex,
Polyacrylamidomethylbenzylidenecamphor manufactured under the name "Mexoryl SW" by Chimex.

### Phenylbenzimidazole derivatives :

Phenylbenzimidazolesulphonic acid sold in particular under the trade name "Eusolex 232" by Merck,
Disodium Phenyl Dibenzimidazole Tetrasulphonate sold under the trade name "Neo Heliopan AP" by Symrise.

### Phenylbenzotriazole derivatives :

Drometrizole Trisiloxane sold under the name "Silatrizole" by Rhodia Chimie, Methylenebis(benzotriazolyl)tetramethylbutylphenol sold in solid form under the trade name "Mixxim BB/100" by Fairmount Chemical or in micronized form as an aqueous dispersion under the trade name "Tinosorb M" by CIBA Specialty Chemicals.

### Triazine derivatives :

Bisethylhexyloxyphenol Methoxyphenyl Triazine sold under the trade name "Tinosorb S" by CIBA Geigy, Ethylhexyl triazone sold in particular under the trade name "Uvinul T150" by BASF,
Diethylhexyl Butamido Triazone sold under the trade name "Uvasorb HEB" by Sigma 3V,
2,4,6-tris(dineopentyl 4'-aminobenzalmalonate)-s-triazine,
2,4,6-tris(diisobutyl 4'-aminobenzalmalonate)-s-triazine,
2,4-bis(n-butyl 4'-aminobenzoate)-6-(aminopropyltrisiloxane)-s-triazine,
2,4-bis(dineopentyl 4'-aminobenzalmalonate)-6-(n-butyl 4'-aminobenzoate)-s-triazine,
the symmetrical triazine screens described in patent US 6,225,467, application WO 2004/085412 (see compounds 6 and 9) or the document "Symmetrical Triazine Derivatives" IP.COM Journal, IP.COM INC WEST HENRIETTA, NY, US (20 September 2004), especially 2,4,6-tris(biphenyl)-1,3,5-triazines (in particular 2,4,6-tris(biphenyl-4-yl-1,3,5-triazine) and 2,4,6-tris(terphenyl)-1,3,5-triazine which is also mentioned in patent applications WO06/035000, WO06/034982, WO06/034991, WO06/035007, WO2006/034992 and WO2006/034985.

### Anthranilic derivatives :

Methyl anthranilate sold under the trade name "Neo Heliopan MA" by Haarmann and Reimer.

### Imidazoline derivatives :

Ethylhexyl Dimethoxybenzylidene Dioxoimidazoline Propionate.

### Benzalmalonate derivatives :

Polyorganosiloxane comprising benzalmalonate functions, such as the Polysilicone-15 sold under the trade name "Parsol SLX" by DSM Nutritional Products Inc..

### 4,4-Diarylbutadiene derivatives :

1,1-dicarboxy(2,2'-dimethylpropyl)-4,4-diphenylbutadiene.

### Benzoxazole derivatives :

2,4-bis[5-1(dimethylpropyl)benzoxazol-2-yl(4-phenyl)imino]-6-(2-ethylhexyl)imino-1,3,5-triazine sold under the name Uvasorb K2A by Sigma 3V.

### Merocyanin derivatives :

Octyl 5-N,N-diethylamino-2-phenylsulphonyl-2,4-pentadienoate and mixtures thereof.

The preferred organic screens are chosen from: ethylhexyl methoxycinnamate,
ethylhexyl salicylate,
homosalate,
butylmethoxydibenzoylmethane,
octocrylene,
phenylbenzimidazolesulphonic acid,
benzophenone-3,
benzophenone-4,
benzophenone-5,
n-hexyl 2-(4-diethylamino-2-hydroxybenzoyl)benzoate, 4-methylbenzylidenecamphor,
terephthalylidenedicamphorsulphonic acid, disodium phenyl dibenzimidazole tetrasulphonate, methylenebis(benzotriazolyl)tetramethylbutylphenol, bisethylhexyloxyphenol methoxyphenyl triazine, ethylhexyl triazone, diethylhexyl butamido triazone,
2,4,6-tris(dineopentyl 4'-aminobenzalmalonate)-s-triazine, 2,4,6-tris(diisobutyl 4'-aminobenzalmalonate)-s-triazine,
2,4-bis(n-butyl 4'-aminobenzoate)-6-(aminopropyltrisiloxane)-s-triazine,
2,4-bis(dineopentyl 4'-aminobenzalmalonate)-6-(n-butyl 4'-aminobenzoate)-s-triazine,
drometrizole trisiloxane,
polysilicone-15,
1,1-dicarboxy(2,2'-dimethylpropyl)-4,4-diphenylbutadiene,
2,4-bis[5-1(dimethylpropyl)benzoxazole-2-yl(4-phenyl)imino]-6-(2-ethylhexyl)imino-1,3,5-triazine, octyl 5-N,N-diethylamino-2-phenylsulphonyl-2,4-pentadienoate,
and mixtures thereof.

The organic UV screens are generally present in the compositions according to the invention in proportions ranging from 0.01% to 20% by weight relative to the total weight of the composition, and preferably ranging from 0.1% to 10% by weight relative to the total weight of the composition.

### (ii) inorganic screens

The inorganic photoprotective agents are chosen from coated or uncoated metal oxide pigments (average primary particle size of: generally between 5 nm and 100 nm, preferably between 10 nm and 50 nm), for instance pigments of titanium oxide (amorphous or crystalline in rutile and/or anatase form), of iron oxide, of zinc oxide, of zirconium oxide or of cerium oxide, which are all UV photoprotective agents well known per se.

The pigments may be coated or uncoated.

The coated pigments are pigments that have undergone one or more surface treatments of chemical, electronic, mechanochemical and/or mechanical nature with compounds as described, for example, in Cosmetics & Toiletries, February 1990, Vol. 105, p. 53-64, such as amino acids, beeswax, fatty acids, fatty alcohols, anionic surfactants, lecithins, sodium, potassium, zinc, iron or aluminium salts of fatty acids, metal alkoxides (of titanium or of aluminium), polyethylene, silicones, proteins (collagen, elastin), alkanolamines, silicon oxides, metal oxides or sodium hexametaphosphate.

As is known, silicones are organosilicon polymers or oligomers of linear or cyclic, branched or crosslinked structure, of variable molecular weight, obtained by polymerization and/or polycondensation of suitably functionalized silanes, and constituted essentially of a repetition of main units in which the silicon atoms are linked together via oxygen atoms (siloxane bond), optionally substituted hydrocarbon-based radicals being directly attached via a carbon atom to said silicon atoms.

The term "silicones" also encompasses the silanes required for their preparation, in particular alkyl silanes.

The silicones used for coating the pigments that are suitable for the present invention are preferably chosen from the group containing alkyl silanes, polydialkylsiloxanes and polyalkylhydrogenosiloxanes. Even more preferably, the silicones are chosen from the group containing octyltrimethylsilane, polydimethylsiloxanes and polymethylhydrogenosiloxanes.

Of course, before being treated with silicones, the metal oxide pigments may have been treated with other surface agents, in particular with cerium oxide, alumina, silica, aluminium compounds or silicon compounds, or mixtures thereof.

The coated pigments are more particularly titanium oxides that have been coated:
with silica, such as the product "Sunveil" from the company Ikeda,
with silica and iron oxide, such as the product "Sunveil F" from the company Ikeda,
with silica and alumina, such as the products "Microtitanium Dioxide MT 500 SA" and "Microtitanium Dioxide MT 100 SA" from the company Tayca, "Tioveil" from the company Tioxide,
with alumina, such as the products "Tipaque TTO-55 (B)" and "Tipaque TTO-55 (A)" from the company Ishihara, and "UVT 14/4" from the company Kemira,
with alumina and aluminium stearate, such as the products "Microtitanium Dioxide MT 100 T, MT 100 TX, MT 100 Z, MT-01" from the company Tayca, the products "Solaveil CT-10 W" and "Solaveil CT 100" from the company Uniqema and the product "Eusolex T-AVO" from the company Merck,
with silica, alumina and alginic acid, such as the product "MT-100 AQ" from the company Tayca,
with alumina and aluminium laurate, such as the product "Microtitanium Dioxide MT 100 S" from the company Tayca,
with iron oxide and iron stearate such as the product "Microtitanium Dioxide MT 100 F" from the company Tayca,
with zinc oxide and zinc stearate, such as the product "BR 351" from the company Tayca,
with silica and alumina and treated with a silicone, such as the products "Microtitanium Dioxide MT 600 SAS", "Microtitanium Dioxide MT 500 SAS" or "Microtitaniuim Dioxide MT 100 SAS" from the company Tayca,
with silica, alumina and aluminium stearate and treated with a silicone, such as the product "STT-30-DS" from the company Titan Kogyo,
with silica and treated with a silicone, such as the product "UV-Titan X 195" from the company Kemira,
with alumina and treated with a silicone, such as the products "Tipaque TTO-55 (S)" from the company Ishihara, or "UV Titan M 262" from the company Kemira, with triethanolamine such as the product "STT-65-S" from the company Titan Kogyo,
with stearic acid such as the product "Tipaque TTO-55 (C)" from the company Ishihara,
with sodium hexametaphosphate, such as the product "Microtitanium Dioxide MT 150 W" from the company Tayca,
the TiO₂ treated with octyltrimethylsilane, sold under the trade name "T 805" by the company Degussa Silices, the TiO₂ treated with a polydimethylsiloxane, sold under the trade name "70250 Cardre UF TiO2SI3" by the company Cardre,
the anatase/rutile TiO₂ treated with a polydimethylhydrogenosiloxane, sold under the trade name "Microtitanium Dioxide USP Grade Hydrophobic" by the company Color techniques.

The uncoated titanium oxide pigments are sold, for example, by the company Tayca under the trade names "Microtitanium Dioxide MT 500 B" or "Microtitanium Dioxide MT600 B" by the company Degussa under the name "P 25", by the company Wacker under the name "Transparent titanium oxide PW", by the company Miyoshi Kasei under the name "UFTR", by the company Tomen under the name "ITS" and by the company Tioxide under the name "Tioveil AQ".

The uncoated zinc oxide pigments are, for example: those sold under the name "Z-cote" by the company Sunsmart;
those sold under the name "Nanox" by the company Elementis;
those sold under the name "Nanogard WCD 2025" by the company Nanophase Technologies.

The coated zinc oxide pigments are, for example:
those sold under the name "Oxide zinc CS-5" by the company Toshibi (ZnO coated with polymethylhydrogenosiloxane);
those sold under the name "Nanogard Zinc Oxide FN" by the company Nanophase Technologies (as a 40% dispersion in Finsolv TN, C₁₂-C₁₅ alkyl benzoate) ;
those sold under the name "Daitopersion ZN-30" and "Daitopersion Zn-50" by the company Daito (dispersions in oxyethylenated cyclopolymethylsiloxane/polydimethylsiloxane, containing 30% or 50% of nanozinc oxides coated with silica and polymethylhydrogenosiloxane);
those sold under the name "NFD Ultrafine ZnO" by the company Daikin (ZnO coated with perfluoroalkyl phosphate and copolymer based on perfluoroalkylethyl as a dispersion in cyclopentasiloxane);
those sold under the name "SPD-Z1" by the company Shin-Etsu (ZnO coated with silicone-grafted acrylic polymer, dispersed in cyclodimethylsiloxane);
those sold under the name "Escalol Z100" by the company ISP (alumina-treated ZnO dispersed in an ethylhexyl methoxycinnamate/copolymer of PVP-hexadecene/methicone mixture);
those sold under the name "Fuji ZnO-SMS-10" by the company Fuji Pigment (ZnO coated with silica and polymethylsilsesquioxane);
those sold under the name "Nanox Gel TN" by the company Elementis (ZnO dispersed at a concentration of 55% in C₁₂-C₁₅ alkyl benzoate with hydroxystearic acid polycondensate).

The uncoated cerium pigments are sold under the name "Colloidal Cerium Oxide" by the company Rhône Poulenc.

The uncoated iron oxide pigments are sold, for example, by the company Arnaud under the names "Nanogard WCD 2002 (FE 45B)", "Nanogard Iron FE 45 BL AQ", "Nanogard FE 45R AQ", "Nanogard WCD 2006 (FE 45R)", or by the company Mitsubishi under the name "TY-220".

The coated iron oxide pigments are, for example, sold by the company Arnaud under the names "Nanogard WCD 2008 (FE 45B FN)", "Nanogard WCD 2009 (FE 45B 556)", "Nanogard FE 45 BL 345", "Nanogard FE 45 BL", or by the company BASF under the name "Transparent Iron Oxide".

Mention may also be made of mixtures of metal oxides, in particular of titanium dioxide and of cerium dioxide, including the silica-coated equal-weight mixture of titanium dioxide and cerium dioxide, sold by the company Ikeda under the name "Sunveil A", and also the alumina-, silica- and silicone-coated mixture of titanium dioxide and of zinc dioxide such as the product "M 261" sold by the company Kemira, or the alumina-, silica- and glycerol-coated mixture of titanium dioxide and of zinc dioxide, such as the product "M 211" sold by the company Kemira.

According to the invention, the coated or uncoated titanium oxide pigments are particularly preferred.

The mineral screens in accordance with the invention represent in general from 0.5% to 40%, preferably from 1% to 30%, of the total weight of the composition.

Depending on their more or less predominant lipophilic, or on the contrary hydrophilic, nature, the pigments may be present either in the fatty phase of the emulsion or in the aqueous phase, or alternatively even in both phases at once.

The aqueous compositions in accordance with the present invention may also comprise conventional cosmetic adjuvants chosen in particular from fatty substances, organic solvents, demulcents, humectants, opacifiers, stabilizers, emollients, silicones, antifoams, fragrances, preserving agents, anionic, cationic, nonionic, zwitterionic or amphoteric surfactants, active agents, fillers, polymers, propellants, acidifying or basifying agents or any other ingredient normally used in the cosmetics and/or dermatological field.

The fatty substances may be constituted of an oil or a wax other than the apolar waxes as defined above, or mixtures thereof. The term "oil" is intended to mean a compound that is liquid at ambient temperature. The term "wax" is intended to mean a compound that is solid or substantially solid at ambient temperature, and the melting point of which is generally above 35°C.

Oils that may be mentioned include mineral oils (paraffin); plant oils (sweet almond oil, macadamia oil, blackcurrant seed oil or jojoba oil); synthetic oils, for instance perhydrosqualene, fatty alcohols or fatty amides (for instance isopropyl lauroyl sarcosinate sold under the name "Eldew SL-205" by the company Ajinomoto), fatty acids or fatty esters, for instance the C₁₂-C₁₅ alkyl benzoate sold under the trade name "Finsolv TN" or "Witconol TN" by the company Witco, 2-ethylphenyl benzoate, for instance the product sold under the name X-TEND 226^{®} by the company ISP, octyl palmitate, isopropyl lanolate, triglycerides, including capric/caprylic acid triglycerides, and dicaprylyl carbonate sold under the name "Cetiol CC" by the company Cognis, oxyethylenated or oxypropylenated fatty esters and ethers; silicone oils (cyclomethicone, polydimethylsiloxanes or PDMS) or fluoro oils, polyalkylenes, and trialkyl trimellitates such as tridecyl trimellitate.

Waxy compounds that may be mentioned include carnauba wax, beeswax, hydrogenated castor oil, polyethylene waxes and polymethylene waxes, for instance the product sold under the name Cirebelle 303 by the company Sasol.

The oily phase of the emulsion preferably represents from 0.1% to 45%, and in particular from 5% to 30%, of the total weight of the liquid phase.

Of course, the fatty phase may also contain one or more conventional lipophilic cosmetic adjuvants, in particular those that are already customarily used in the manufacture and production of cosmetic antisun compositions.

Conventionally, the continuous aqueous phase may be constituted of water, or a mixture of water and polyhydric alcohol(s), for instance glycerol, sorbitol or alkanediols such as caprylyl glycol; ethers of a glycol such as ethylene glycol, propylene glycol, butylene glycol, dipropylene glycol or diethylene glycol, or else a mixture of water and of water-soluble lower alcohol(s) such as ethanol, isopropanol or butanol, and it may, of course, also contain conventional water-soluble cosmetic adjuvants.

Of course, those skilled in the art will take care to select the possible additional compound(s) mentioned above and/or the amounts thereof in such a way that the advantageous properties intrinsically associated with the compositions in accordance with the invention are not, or not substantially, impaired by the addition(s) envisaged, in particular the stability of the emulsion over time and its effectiveness.

The compositions according to the invention may be prepared according to the techniques well known to those skilled in the art, such as a fluid cream, a milk or a lotion. They may optionally be packaged in an aerosol, and may be in the form of a mousse, a spray or a mister.

The compositions according to the invention are of use in a large number of treatments, in particular cosmetic treatments, of the skin, the lips and the hair, including the scalp, in particular for protecting and/or caring for the skin, the lips and/or the hair, and/or for making up the skin and/or the lips.

Another subject of the present invention comprises the use of the compositions according to the invention as defined above, for the manufacture of products for the cosmetic treatment of the skin, the lips, the nails, the hair, the eyelashes, the eyebrows and/or the scalp, in particular care products, antisun products and makeup products.

The cosmetic compositions according to the invention may, for example, be used as a makeup product.

The cosmetic compositions according to the invention may, for example, be used as a care product and/or antisun product for the face and/or the body, which has a fluid liquid consistency. They may optionally be packaged in an aerosol and may be in the form of a mousse, a spray or a mister.

The compositions according to the invention in the form of sprayable fluid lotions in accordance with the invention may be applied to the skin or the hair in the form of fine particles by means of pressurization devices. The devices in accordance with the invention are well known to those skilled in the art and comprise nonaerosol pump dispensers or "atomizers", aerosol containers comprising a propellant and also aerosol pump dispensers using compressed air as propellant. The latter are described in patents US 4,077,441 and US 4,850 517.

The compositions packaged as an aerosol in accordance with the invention generally contain conventional propellants such as, for example, the hydrofluorinated compounds dichlorodifluoromethane, difluoroethane, dimethyl ether, isobutane, n-butane, propane or trichlorofluoromethane. They are preferably present in amounts ranging from 15% to 50% by weight relative to the total weight of the composition.

A subject of the invention is also a process for preparing such emulsified photoprotective compositions, comprising the homogenization, at a temperature of between 40 and 70°C and using a rotor-stator type homogenizer or mixer, of a fatty phase containing optionally at least one gemini surfactant and optionally at least one organic UV screen and/or one mineral UV screen, and of an aqueous phase containing optionally one gemini surfactant, optionally one or more organic UV screen(s) and optionally at least one organic UV screen and/or one mineral UV screen, it being understood that at least one of these phases contains a gemini surfactant, a crosslinked copolymer of methacrylic acid and of C₁-C₄ alkyl acrylate being incorporated either into the aqueous phase or the fatty phase before homogenization, or into the fine emulsion obtained, after cooling to a temperature of between 30 and 40°C.

The organic or mineral UV screen (s) in solid form may also be incorporated into the oil-in-water emulsion containing the crosslinked copolymer of methacrylic acid and of C₁-C₄ alkyl acrylate. In this case, it is necessary to subject the composition to a second homogenization step.

The process of the present invention stands out by virtue of the fact that it can be carried out without any high-pressure homogenization step.

The compositions according to the invention may also further comprise additional cosmetic and dermatological active agents.

The additional active agents may in particular be chosen from moisturisizers, desquamating agents, agents for improving the barrier function, depigmenting agents, antioxidants, dermo-decontracting agents, anti-glycation agents, agents for stimulating the synthesis of dermal and/or epidermal macromolecules and/or for preventing their degradation, agents for stimulating fibroblast or keratinocyte proliferation and/or keratinocyte differentiation, agents for promoting the maturation of the horny envelope, NO-synthase inhibitors, peripheral benzodiazepine receptor (PBR) antagonists, agents for increasing sebaceous gland activity, agents for stimulating the energy metabolism of cells, tensioning agents, liporestructuring agents, slimming agents, agents for promoting cutaneous capillary circulation, calmatives and/or anti-irritants, seboregulators or anti-seborrhoeic agents, astringents, cicatrizing agents, anti-inflammatory agents and anti-acne agents.

Those skilled in the art will select said active agent (s) according to the desired effect on the skin, the hair, the eyelashes, the eyebrows or the nails.

For caring for and/or making up elderly skin, those skilled in the art will select, as a preference, at least one active agent chosen from moisturizers, desquamating agents, agents for improving the barrier function, depigmenting agents, antioxidants, dermo-decontracting agents, anti-glycation agents, agents for stimulating the synthesis of dermal and/or epidermal macromolecules and/or for preventing their degradation, agents for stimulating fibroblast or keratinocyte proliferation and/or keratinocyte differentiation, agents for promoting the maturation of the horny envelope, NO-synthase inhibitors, peripheral benzodiazepine receptor (PBR) antagonists, agents for increasing sebaceous gland activity, agents for stimulating the energy metabolism of cells, liporestructuring agents and agents for promoting the cutaneous capillary circulation for the area around the eyes.

The composition may also comprise at least one ingredient such as fillers with a soft-focus effect or agents for promoting the natural colouring of the skin, intended to complete the biological effect of these active agents or to provide an immediate visual anti-aging effect.

For caring for and/or making up greasy skin, those skilled in the art will select, as a preference, at least one active agent chosen from from desquamating agents, sebo-regulators or anti-seborrhoeic agents, and astringents.

The composition may also comprise at least one additional ingredient for completing the biologic effect of these active agents or providing an immediate visual effect; mention may in particular be made of matting agents, fillers with a soft-focus effect, fluorescers, agents for promoting the naturally pinkish coloration of the skin, and abrasive fillers or exfoliants, etc.

### 1. Moisturizers or humectants

Moisturizers or humectants that may especially be mentioned include glycerol and derivatives thereof, urea and derivatives thereof, especially Hydrovance^{®} sold by National Starch, lactic acids, hyaluronic acid, AHAs, BHAs, sodium pidolate, xylitol, serine, sodium lactate, ectoin and derivatives thereof, chitosan and derivatives thereof, collagen, plankton, an extract of *Imperata cylindra* sold under the name Moist 24^{®} by the company Sederma, acrylic acid homopolymers, for instance Lipidure-HM^{®} from NOF Corporation, beta-glucan and in particular sodium carboxymethyl beta-glucan from Mibelle-AG-Biochemistry; a mixture of passionflower oil, apricot oil, corn oil and rice bran oil sold by Nestlé under the name NutraLipids^{®}; a C-glycoside derivative such as those described in patent application WO 02/051 828 and in particular C-β-D-xylopyranoside-2-hydroxypropane in the form of a solution containing 30% by weight of active material in a water/propylene glycol mixture (60/40% by weight) such as the product sold by Chimex under the trade name Mexoryl SBB^{®}; an oil of musk rose sold by Nestlé; an extract of the microalga *Prophyridium cruentum* enriched with zinc, sold by Vincience under the name Algualane Zinc^{®}; spheres of collagen and of chondroitin sulphate of marine origin (Ateocollagen) sold by the company Engelhard Lyon under the name Marine Filling Spheres; hyaluronic acid spheres such as those sold by the company Engelhard Lyon; and arginine.

The moisturizer that will preferably be used is chosen from urea and derivatives thereof, especially Hydrovance^{®} sold by National Starch, hyaluronic acid, AHAs, BHAs, acrylic acid homopolymers, for instance Lipidure-HM^{®} from NOF Corporation, beta-glucan and in particular sodium carboxymethyl beta-glucan from Mibelle-AG-Biochemistry; a mixture of passionflower oil, apricot oil, corn oil and rice bran oil sold by Nestlé under the name NutraLipids^{®}; a C-glycoside derivative such as those described in patent application WO 02/051 828 and in particular C-β-D-xylopyranoside-2-hydroxypropane in the form of a solution containing 30% by weight of active material in a water/propylene glycol mixture (60/40% by weight) such as the product sold by Chimex under the trade name Mexoryl SBB^{®}; an oil of musk rose sold by Nestlé; an extract of the microalga *Prophyridium cruentum* enriched with zinc, sold by Vincience under the name Algualane Zinc^{®}; spheres of collagen and of chondroitin sulphate of marine origin (Ateocollagen) sold by the company Engelhard Lyon under the name Marine Filling Spheres; hyaluronic acid spheres such as those sold by the company Engelhard Lyon; and arginine.

### 2. Desquamating agents

The term "desquamating agent" is intended to mean any compound capable of acting:
- either directly on desquamation by promoting exfoliation, such as β-hydroxy acids (BHA), in particular salicylic acid and derivatives thereof (including 5-n-octanoylsalicylic acid, also known as capryloyl salicylic acid as the INCI name); α-hydroxy acids (AHA), such as glycolic acid, citric acid, lactic acid, tartaric acid, malic acid or mandelic acid; 8-hexadecene-1,16-dicarboxylic acid or 9-octadecenedioic acid; urea and derivatives thereof; gentisic acid and derivatives thereof; oligofucoses; cinnamic acid; *Saphora japonica* extract; resveratrol, and certain jasmonic acid derivatives;
- or on the enzymes involved in the desquamation or degradation of corneodesmosomes, glycosidases, stratum corneum chymotryptic enzyme (SCCE) or other proteases (trypsin, chymotrypsin-like). Mention may be made of aminosulphonic compounds and in particular 4-(2-hydroxyethyl)piperazine-1-propanesulphonic acid (HEPES); 2-oxothiazolidine-4-carboxylic acid (procysteine) and its derivatives; derivatives of α-amino acids of glycine type (as described in EP-0 852 949, and also sodium methyl glycine diacetate sold by BASF under the trade name Trilon M); honey; and derivatives of sugar such as O-octanoyl-6-D-maltose and N-acetylglucosamine.

As other desquamating agents that may be used in the composition according to the invention, mention may be made of:
- oligofructoses, EDTA and derivatives thereof, laminaria extracts, o-linoleyl-6D-glucose, (3-hydroxy-2-pentylcyclopentyl)acetic acid, glycerol trilactate, O-octanyl-6'-D-maltose, S-carboxymethylcysteine, siliceous derivatives of salicylate such as those described in patent EP 0 796 861, oligofucases such as those described in patent EP 0 218 200, 5-acyl salicylic acid salts, active agents with effects on transglutaminase, as in patent EP 0 899 330,
- extract of the flowers of *Ficus opuntia indica* (Exfolactive^{®} from Silab),
- 8-hexadecene-1,16-dicarboxylic acid,
- esters of glucose and of vitamin F, and
- mixtures thereof.

Preferred desquamating agents that may be mentioned include β-hydroxy acids such as 5-n-octanoyl salicylic acid; urea; glycolic acid, citric acid, lactic acid, tartaric acid, malic acid or mandelic acid; 4-(2-hydroxyethyl)piperazine-1-propanesulphonic acid (HEPES); extract of *Saphora japonica*; honey; N-acetyl glucosamine; sodium methyl glycine diacetate, and mixtures thereof.

Even more preferentially, a desquamating agent chosen from 5-n-octanoyl salicylic acid; urea; 4-(2-hydroxyethyl)piperazine-1-propanesulphonic acid (HEPES); extract of *Saphora japonica*; honey; N-acetyl glucosamine; sodium methyl glycine diacetate, and mixtures thereof, will be used in the compositions of the invention.

### 3. Agents for improving the barrier function

As agents for improving the barrier function, mention may be made especially of arginine, serine, an extract of *Thermus thermophilus* such as Venuceane^{®} from Sederma, an extract of the rhizome of wild yam (*Dioscorea villosa*) such as Actigen Y^{®} from Active Organics, plankton extracts, for instance Omega Plankton^{®} from Secma, yeast extracts, for instance Relipidium^{®} from Coletica, a chestnut extract such as Recoverine^{®} from Silab, a cedar bud extract such as Gatuline Zen^{®} from Gattefossé, sphingosines, for instance salicyloyl sphingosine sold under the name Phytosphingosine^{®} SLC by the company Degussa, a mixture of xylitol, polyxylityl glycoside and xylitan, for instance Aquaxyl^{®} from SEPPIC, extracts of Solanacea plants, for instance Lipidessence^{®} from Coletica; omega-3 unsaturated oils such as musk rose oils; and mixtures thereof.

Mention may also be made especially of ceramides or derivatives thereof, in particular ceramides of type 2 (for instance N-oleoyldihydrosphingosine), of type 3 (for instance stearoyl-4-hydroxysphinganine, as the INCI name) and of type 5 (for instance N-2-hydroxypalmitoyldihydrosphingosine, having the INCI name: hydroxypalmitoyl sphinganine), sphingoid-based compounds, glycosphingolipids, phospholipids, cholesterol and derivatives thereof, phytosterols, essential fatty acids, diacylglycerol, 4-chromanone and chromone derivatives, petroleum jelly, lanolin, shea butter, cocoa butter, and PCA salts.

As preferred agents having a restructuring effect on the cutaneous barrier, mention will be made of an extract of *Thermus thermophilus*, an extract of wild yam rhizome (*Dioscorea villosa*), a yeast extract, a chestnut extract, a cedar bud extract, arginine, serine, ceramides especially of type 3 and 5; and mixtures thereof.

Serine or arginine, or a mixture thereof, will preferably be used.

### 4. Depigmenting agents

Depigmenting agents that may especially be mentioned include vitamin C and derivatives thereof and especially vitamin CG, CP and 3-0 ethyl vitamin C, alpha and beta arbutin, ferulic acid, lucinol and derivatives thereof, kojic acid, resorcinol and derivatives thereof, tranexamic acid and derivatives thereof, gentisic acid, homogentisate, methyl gentisate or homogentisate, dioic acid, calcium D-pantheteine sulphonate, lipoic acid, ellagic acid, vitamin B3, linoleic acid and derivatives thereof, ceramides and homologues thereof, plant derivatives, for instance camomile, bearberry, the aloe family (vera, ferox, bardensis), mulberry or skullcap; a kiwi fruit (*Actinidia chinensis*) juice sold by Gattefossé, an extract of *Paeonia suffruticosa* root, such as the product sold by the company Ichimaru Pharcos under the name Botanpi Liquid B^{®}, an extract of brown sugar (*Saccharum officinarum*), such as the extract of molasses sold by the company Taiyo Kagaku under the name Molasses Liquid, without this list being exhaustive.

Vitamin C and derivatives thereof and especially vitamin CG, CP and 3-0 ethyl vitamin C, alpha and beta arbutin, ferulic acid, kojic acid, resorcinol and derivatives thereof, calcium D-pantheteine sulphonate, lipoic acid, ellagic acid, vitamin B3, a kiwi fruit (*Actinidia chinensis*) juice sold by Gattefossé, and an extract of *Paeonia suffruticosa* root, such as the product sold by the company Ichimaru Pharcos under the name Botanpi Liquid B^{®}, will be used as preferred depigmenting agents.

### 5. Antioxidants

Mention may be made especially of tocopherol and esters thereof, in particular tocopherol acetate; ascorbic acid and derivatives thereof, in particular magnesium ascorbyl phosphate and ascorbyl glucoside; ferulic acid; serine; ellagic acid, phloretin, polyphenols, tannins, tannic acid, epigallocathechins and natural extracts containing them, anthocyans, extracts of rosemary, olive leaf extracts, for instance those from the company Silab, extracts of green tea, resveratrol and derivatives thereof, ergothioneine, N-acetylcysteine, an extract of the brown alga *Pelvetia canaliculata*, for instance Pelvetiane^{®} from Secma, chlorogenic acid, biotin, chelating agents, such as BHT, BHA, N,N'-bis(3,4,5-trimethoxybenzyl)ethylenediamine and salts thereof; idebenone, plant extracts, for instance Pronalen Bioprotect TM from the company Provital; coenzyme Q10, bioflavonoids, SODs, phytantriol, lignans, melatonin, pidolates, gluthatione, caprylyl glycol, phloretin, TotarolTM or extract of *Podocarpus totara* containing totarol (totara-8, 11, 13-trienol or 2-phenanthrenol, 4b, 5, 6, 7, 8, 8a, 9, 10-octahydro-4b, 8, 8-trimethyl-1(1-methylethyl)-; an extract of jasmine such as that sold by the company Silab under the name Helisun^{®}; hesperitin laurate such as Flavagrum PEG^{®} from the company Engelhard Lyon; an extract of *Paeonia suffruticosa* root, such as the product sold by the company Ichimaru Pharcos under the name Botanpi Liquid B^{®}; an extract of lychee, such as the extract of lychee pericarp sold by the company Cognis under the name Litchiderm LS 9704^{®}, an extract of pomegranate fruit (*Punica granatum*), such as the product sold by the company Draco Natural products.

Other anti-aging agents that may be mentioned include DHEA and derivatives thereof, boswellic acid, extracts of rosemary, carotenoids (β-carotene, zeaxanthin, lutein), cysteic acid, copper derivatives and jasmonic acid.

Preferred antioxidants that will especially be used include ferulic acid; serine; phloretin, an extract of pomegranate, biotin, chelating agents, such as BHT, BHA, N,N'-bis(3,4,5-trimethoxybenzyl)ethylenediamine and salts thereof, caprylyl glycol, phloretin, TotarolTM, an extract of jasmine such as the product sold by Silab under the name Helisun^{®}; hesperitin laurate, such as Flavagrum PEG^{®} from the company Engelhard Lyon; an extract of *Paeonia suffruticosa* root, such as the product sold by the company Ichimaru Pharcos under the name Botanpi Liquid B^{®}.

### 6. Dermo-relaxing or dermo-decontracting agents

Examples that may be mentioned include manganese gluconate and other salts, adenosine, alverine citrate and salts thereof, glycine, an extract of *Iris pallida*, a hexapeptide (Argeriline R from Lipotec) or sapogenins, for instance wild yam and the carbonyl amines described in application EP1484052. Examples of sapogenins that may be mentioned include those described in patent application WO 02/47650, in particular wild yam, the diosgenin extracted especially from *Dioscorea opposita* or any extract naturally containing or containing after treatment one or more sapogenins (wild yam rhizome, agave leaf, which contains hecogenin and tigogenin, extracts of Liliacea plants and more particularly Yucca or Smilax containing smilagenin and sarsapogenin, or salsepareilla root) or Actigen Y from the company Active Organics; or ginger. Mention may also be made of DMAE (dimethyl MEA), extracts of sea fennel, of rockrose, of helicrysum, of aniseed and of paracress, and an extract of *Acmella oleracea*, for instance Gatuline^{®} from Gattefossé.

Preferred dermo-relaxing agents that will be mentioned include adenosine, manganese gluconate, wild yam, sea fennel, glycine and alverine.

### 7. Anti-glycation agents

The term "anti-glycation agent" means a compound that prevents and/or reduces the glycation of skin proteins, in particular dermal proteins such as collagen. Anti-glycation agents that may especially be mentioned include extracts of plants of the Ericacea family, such as an extract of blueberry (*Vaccinium angustifolium* or *Vaccinium myrtillus*), for example the product sold under the name Blueberry Herbasol Extract PG by the company Cosmetochem, ergothioneine and derivatives thereof, hydroxystilbenes and derivatives thereof, such as resveratrol and 3,3',5,5'-tetrahydroxystilbene (these anti-glycation agents are described in patent applications FR 2 802 425, FR 2 810 548, FR 2 796 278 and FR 2 802 420, respectively), dihydroxystilbenes and derivatives thereof, polypeptides of arginine and of lysine such as the product sold under the name Amadorine^{®} by the company Solabia, carcinine hydrochloride (sold by Exsymol under the name Alistin^{®}), an extract of *Helianthus annuus*, for instance Antiglyskin^{®} from Silab, wine extracts such as the extract of powdered white wine on a maltodextrin support sold under the name Vin blanc déshydraté 2F by the company Givaudan, thioctic acid (or alpha-lipoic acid), a mixture of extract of bearberry and of marine glycogen, for instance Aglycal LS 8777^{®} from Laboratoires Sérobiologiques, and an extract of black tea, for instance Kombuchka^{®} from Sederma, and mixtures thereof.

Preferred anti-glycation agents that will be mentioned include extracts of blueberry (*Vaccinium myrtillus*) and extracts of black tea.

### 8. Agents for stimulating the synthesis of dermal and/or epidermal macromolecules and/or for preventing their degradation

Among the active agents for stimulating the dermal macromolecules or for preventing their degradation, mention may be made of those acting:
- either on collagen synthesis, such as extracts of *Centella asiatica*, asiaticosides and derivatives thereof; ascorbic acid or vitamin C and derivatives thereof; synthetic peptides such as iamin, biopeptide CL or palmitoyloligopeptide sold by the company Sederma; peptides extracted from plants, such as the soybean hydrolysate sold by the company Coletica under the trade name Phytokine^{®}; rice peptides such as Nutripeptide^{®} from Silab, methylsilanol mannuronate such as Algisium C^{®} sold by Exsymol; plant hormones such as auxins and lignans; folic acid; and an extract of *Medicago sativa* (alfalfa) such as the product sold by Silab under the name Vitanol^{®}; a peptide extract of hazelnut such as the product sold by the company Solabia under the name Nuteline C^{®}; and arginine;
- or on the inhibition of collagen degradation, in particular agents acting on the inhibition of metalloproteases (MMP) more particularly such as MMP 1, 2, 3 and 9. Mention may be made of: retinoids and derivatives, extracts of *Medicago sativa* such as Vitanol^{®} from Silab, an extract of *Aphanizomenon flosaquae* (Cyanophyceae) sold under the name Lanablue^{®} by Atrium Biotechnologies, oligopeptides and lipopeptides, lipoamino acids, the malt extract sold by the company Coletica under the trade name Collalift^{®}; blueberry or rosemary extracts; lycopene; isoflavones, derivatives thereof or plant extracts containing them, in particular extracts of soybean (sold, for example, by the company Ichimaru Pharcos under the trade name Flavosterone SB^{®}), of red clover, of flax or of kakkon; an extract of lychee such as the lychee pericarp extract sold by the company Cognis under the trade name Litchiderm LS 9704^{®}; Dipalmitoyl Hydroxyproline sold by SEPPIC under the name Sepilift DPHP^{®}: *Baccharis genistelloides* or Baccharine sold by Silab, an extract of moringa such as Arganyl LS 9781^{®} from Cognis; the sage extract described in patent application FR-A-2 812 544 from the Labiatae family (*Salvia officinalis* from the company Flacksmann), an extract of rhododendron, a blueberry extract, and an extract of *Vaccinium myrtillus* such as those described in patent application FR-A-2 814 950;
- or on the synthesis of molecules belonging to the elastin family (elastin and fibrillin), such as:
   retinol and derivatives, in particular retinol palmitate; the extract of *Saccharomyces cerevisiae* sold by the company LSN under the trade name Cytovitin^{®}; and
   the extract of the alga *Macrocystis pyrifera* sold by the company Secma under the trade name Kelpadelie^{®}; a peptide extract of hazelnut such as the product sold by the company Solabia under the trade name Nuteline C^{®};
- or on the inhibition of elastin degradation, such as the peptide extract of seeds of *Pisum sativum* sold by the company LSN under the trade name Parelastyl^{®}; heparinoids; and the N-acylamino amide compounds described in patent application WO 01/94381, such as {2-[acetyl(3-trifluoromethylphenyl)amino]-3-methyl-butyrylamino}acetic acid, also known as N-[N-acetyl, N'-(3-trifluoromethyl)phenylvalyl]glycine, or N-acetyl-N-[3-(trifluoromethyl)phenyl]valyl glycine or acetyl trifluoromethyl phenyl valylglycine, or an ester thereof with a C₁-C₆ alcohol; an extract of rice peptides such as Colhibin^{®} from Pentapharm, or an extract of *Phyllanthus emblica such as* Emblica^{®} from Rona;
- or on the synthesis of glycosaminoglycans, such as the product of fermentation of milk with *Lactobacillus vulgaris*, sold by the company Brooks under the trade name Biomin yogourth^{®}; the extract of the brown alga *Padina pavonica* sold by the company Alban Muller under the trade name HSP3^{®}; the *Saccharomyces cerevisiae* extract available especially from the company Silab under the trade name Firmalift^{®} or from the company LSN under the trade name Cytovitin^{®}; an extract of *Laminaria ochroleuca* such as Laminaine^{®} from Secma; essence of Mamaku from Lucas Meyer, and an extract of cress (Odraline^{®} from Silab);
- or on the synthesis of fibronectin, such as the extract of the zooplankton Salina sold by the company Seporga under the trade name GP4G^{®};
the yeast extract available especially from the company Alban Muller under the trade name Drieline^{®}; and the palmitoyl pentapeptide sold by the company Sederma under the trade name Matrixil^{®}.

Among the active agents for stimulating epidermal macromolecules, such as fillagrin and keratins, mention may be made especially of the extract of lupin sold by the company Silab under the trade name Structurine^{®}; the extract of *Fagus sylvatica* beech buds sold by the company Gattefossé under the trade name Gatuline^{®} RC; and the extract of the zooplankton Salina sold by the company Seporga under the trade name GP4G^{®}; the copper tripeptide from Procyte; a peptide extract of *Voandzeia substerranea*, such as the product sold by the company Laboratoires Sérobiologiques under the trade name Filladyn LS 9397^{®}.

Preferably, an active agent that stimulates the synthesis of dermal and/or epidermal macromolecules and/or that prevents their degradation, chosen from agents for stimulating the synthesis of glycosaminoglycans, agents for inhibiting elastine degradation, agents for stimulating fibronectin synthesis, agents for stimulating the synthesis of epidermal macromolecules, and mixtures thereof, will preferably be used.

Even more preferentially, an active agent that stimulates the synthesis of glycosaminogylcans, chosen from an extract of the brown alga *Padina pavonica*, an extract of *Saccharomyces cerevisiae*, an extract of *Laminaria ochroleuca*, essence of Mamaku, an extract of cress, and mixtures thereof, will be used.

As preferred active agents for stimulating the synthesis of dermal and/or epidermal macromolecules and/or for preventing their degradation, mention may be made of:
synthetic peptides such as iamin, the biopeptide CL or palmitoyloligopeptide sold by the company Sederma; peptides extracted from plants, such as the soybean hydrolysate sold by the company Coletica under the trade name Phytokine^{®}; rice peptides such as Nutripeptide^{®} from Silab, methylsilanol mannuronate such as Algisium C^{®} sold by Exsymol; folic acid, an extract of *Medicago sativa* (alfafa), such as the product sold by Silab under the name Vitanol^{®}; a peptide extract of hazelnut, such as the product sold by the company Solabia under the name Nuteline C^{®}; arginine; an extract of *Aphanizomenon flos-aquae* (Cyanophyceae) sold under the name Lanablue^{®} by Atrium Biotechnologies, the malt extract sold by the company Coletica under the trade name Collalift^{®}; lycopene; an extract of lychee; an extract of moringa, such as Arganyl LS 9781^{®} from Cognis; an extract of *Vaccinium myrtillus* such as those described in patent application FR-A-2814950; retinol and derivatives, in particular retinol palmitate; the extract of *Saccharomyces cerivisiae* sold by the company LSN under the trade name Cytovitin^{®}; a peptide extract of hazelnut such as the product sold by the company Solabia under the name Nuteline C^{®}; {2-[acetyl(3-trifluoromethylphenyl)amino]-3-methylbutyrylamino}acetic acid, also known as N-[N-acetyl-N'-(3-trifluoromethyl)phenylvalyl]glycine or N-acetyl-N-[3-(trifluoromethyl)phenyl]valylglycine or acetyl trifluoromethyl phenyl valylglycine, or an ester thereof with a C₁-C₆ alcohol; an extract of rice peptides such as Colhibin^{®} from Pentapharm, or an extract of *Phyllanthus emblica* such as Emblica^{®} from Rona; the extract of the brown alga *Padina pavonica* sold by the company Alban Muller under the trade name HSP3^{®}; the extract of *Saccharomyces cerevisiae* available especially from the company Silab under the trade name Firmalift^{®} or from the company LSN under the trade name Cytovitin^{®}; an extract of *Laminaria ochroleuca* such as Laminaine^{®} from Secma; essence of Mamaku from Lucas Meyer, the extract of lupin sold by the company Silab under the trade name Structurine^{®}; the extract of *Fagus sylvatica* beech buds sold by the company Gattefossé under the trade name Gatuline^{®} RC.

### 9. Agents for stimulating fibroblast or keratinocyte proliferation and/or keratinocyte differentiation

The agents for stimulating fibroblast proliferation that may be used in the composition according to the invention may be chosen, for example, from plant proteins or polypeptides, extracted especially from soybean (for example a soybean extract sold by the company LSN under the name Eleseryl SH-VEG 8^{®} or sold by the company Silab under the trade name Raffermine^{®}); an extract of hydrolysed soybean proteins such as Ridulisse^{®} from Silab; and plant hormones such as gibberellins and cytokinins; a peptide extract of hazelnut such as the product sold by the company Solabia under the name Nuteline C^{®}.

Preferably, an agent that promotes keratinocyte proliferation and/or differentiation will be used.

The agents for stimulating keratinocyte proliferation that may be used in the composition according to the invention especially comprise adenosine; phloroglucinol, the extract of *Hydrangea macrophylla* leaves, for instance Amacha Liquid E^{®} from Ichimaru Pharcos, a yeast extract such as Stimoderm^{®} from CLR; the extract of *Larrea divaricata* such as Capislow^{®} from Sederma, mixtures of extract of papaya, of olive tree leaves and of lemon, such as Xyleine^{®} from Vincience, the extract of *Hydrangea macrophylla* leaves, for instance Amacha Liquid E^{®} from Ichimaru Pharcos, retinol and esters thereof, including retinyl palmitate, phloroglucinol, the nut cake extracts sold by Gattefossé and the extracts of *Solanum tuberosum* such as Dermolectine^{®} sold by Sederma.

Among the agents for stimulating keratinocyte differentiation are, for example, minerals such as calcium; sea fennel, a peptide extract of lupin, such as the product sold by the company Silab under the trade name Structurine^{®}; sodium beta-sitosteryl sulphate, such as the product sold by the company Seporga under the trade name Phytocohesine^{®}; and a water-soluble extract of corn, such as the product sold by the company Solabia under the trade name Phytovityl^{®}; a peptide extract of *Voandzeia substerranea* such as the product sold by the company Laboratoires Sérobiologiques under the trade name Filladyn LS 9397^{®}; and lignans such as secoisolariciresinol, and retinol and esters thereof, including retinyl palmitate.

As agents for stimulating keratinocyte proliferation and/or differentiation, mention may also be made of oestrogens such as oestradiol and homologues; and cytokines.

As preferred active agents for stimulating fibroblast or keratinocyte proliferation and/or keratinocyte differentiation, mention will be made of plant proteins or polypeptides, extracted especially from soybean (for example a soybean extract sold by the company LSN under the name Eleseryl SH-VEG 8^{®} or sold by the company Silab under the trade name Raffermine^{®}); an extract of hydrolysed soybean proteins such as Ridulisse^{®} from Silab; a peptide extract of hazelnut such as the product sold by the company Solabia under the name Nuteline C^{®}; adenosine; phloroglucinol, a yeast extract such as Stimoderm^{®} from CLR; a peptide extract of lupin such as the product sold by the company Silab under the trade name Structurine^{®}; a water-soluble corn extract, such as the product sold by the company Solabia under the trade name Phytovityl^{®}; a peptide extract of *Voandzeia substerranea*, such as the product sold by the company Laboratoires Sérobiologiques under the trade name Filladyn LS 9397^{®}; retinol and esters thereof, including retinyl palmitate.

### 10. Agents for promoting the maturation of the horny envelope

Agents that participate in the maturation of the horny envelope, which becomes impaired with age and induces a decrease in transglutaminase activity, may be used in the compositions of the invention. Examples that may be mentioned include urea and derivatives thereof and in particular Hydrovance^{®} from National Starch and the other active agents mentioned in L'Oréal patent application FR 2 877 220 (unpublished).

### 11. NO-synthase inhibitors

The agent with an inhibitory action on NO-synthase may be chosen from OPCs (oligomeric proanthocyanidins); plant extracts of the species *Vitis vinifera* sold especially by the company Euromed under the name "Leucocyanidines de raisins extra", or by the company Indena under the name Leucoselect^{®}, or finally by the company Hansen under the name "Extrait de marc de raisin"; plant extracts of the species *Olea europaea* preferably obtained from olive tree leaves and sold especially by the company Vinyals in the form of a dry extract, or by the company Biologia & Technologia under the trade name Eurol^{®} BT; and plant extracts of the species *Gingko biloba*, preferably a dry aqueous extract of this plant sold by the company Beaufour under the trade name "Ginkgo biloba extrait standard", and mixtures thereof.

### 12. Peripheral benzodiazepine receptor (PBR) antagonists

Mention may be made, for example, of 1-(2-chlorophenyl)-N-(1-methylpropyl)-3-isoquinoline carboxamide; the compounds described in patent applications WO 03/030 937 and WO 03/068 753, pyridazino[4,5-b]indole-1-acetamide derivatives of general formula (VII) as described in document WO 00/44384.

### 13. Agents for increasing sebaceous gland activity

Mention may be made, for example, of methyl dehydrojasmonate, hecogenin, hedione and O-linoleyl-6D-glucose, and mixtures thereof.

### 14. Agents for stimulating the energy metabolism of cells

The active agent for stimulating the energy metabolism of cells may be chosen, for example, from biotin, an extract of *Saccharomyces cerevisiae* such as Phosphovital^{®} from Sederma, the mixture of sodium, manganese, zinc and magnesium salts of pyrrolidonecarboxylic acid, for instance Physiogenyl^{®} from Solabia, a mixture of zinc, copper and magnesium gluconate, such as Sepitonic M3^{®} from SEPPIC, and mixtures thereof; a beta-glucan derived from *Saccharomyces cerevisiae*, such as the product sold by the company Mibelle AG Biochemistry.

### 15. Tensioning agents

The term "tensioning agent" that may be used according to the invention means compounds liable to have a tensioning effect, i.e. being able to make the skin taut.

According to the invention, the term "tensioning agent" generally means any compound that is soluble or dispersible in water at a temperature ranging from 25°C to 50°C at a concentration of 7% by weight in water or at the maximum concentration at which a medium of uniform appearance is formed and producing at this concentration of 7% or at this maximum concentration in water a shrinkage of more than 15% in the test described below.

The maximum concentration at which a medium of uniform appearance forms is determined to within ± 10% and preferably to within ± 5%.

The expression "medium of uniform appearance" means a medium that does not contain any aggregates that are visible to the naked eye.

For the determination of said maximum concentration, the tensioning agent is gradually added to the water with deflocculating stirring at a temperature ranging from 25°C to 50°C, and the mixture is then stirred for one hour. The mixture thus prepared is then examined after 24 hours to see if it is of uniform appearance (absence of aggregates visible to the naked eye).

The tensioning effect may be characterized by an *in vitro* shrinkage test.
A homogeneous mixture of the tensioning agent in water, at a concentration of 7% by weight or at the maximum concentration defined above, is prepared beforehand and as described previously. 30 µl of the homogeneous mixture are placed on a rectangular sample (10 × 40 mm, thus having an initial width L₀ of 10 mm) of elastomer with a modulus of 20 MPa and a thickness of 100 µm.
After drying for 3 hours at 22 ± 3°C and 40 ± 10% relative humidity RH, the elastomer sample has a shrunken width, denoted L₃ₕ, due to the tension exerted by the applied tensioning agent.

The tensioning effect (TE) of said agent is then quantified in the following manner:
"TE" = (L₀ - L₃ₕ/L₀) × 100 as %
with L₀ = initial width 10 mm
and L₃ₕ = width after 3 hours of drying

The tensioning agent may be chosen from:
plant or animal proteins and hydrolysates thereof;
polysaccharides of natural origin;
mixed silicates;
colloidal particles of inorganic fillers;
synthetic polymers;
and mixtures thereof.

Those skilled in the art will know how to select, from the chemical categories listed above, the materials that satisfy to the tensioning test as described above.

Mention may especially be made of:
(a) plant proteins and protein hydrolysates, in particular of corn, rye, wheat, buckwheat, sesame, spelt, pea, bean, lentil, soybean and lupin,
(b) polysaccharides of natural origin, especially (a) polyholosides, for example (i) in the form of starch derived especially from rice, corn, potato, cassava, pea, wheat, oat, etc. or (ii) in the form of carrageenans, alginates, agars, gellans, cellulose polymers and pectins, advantageously as an aqueous dispersion of gel microparticles, and (b) latices consisting of shellac resin, sandarac gum, dammar resins, elemi gums, copal resins, cellulose derivatives, and mixtures thereof,
(c) mixed silicates, especially phyllosilicates and in particular Laponites,
(d) colloidal particles of mineral fillers with a number-average diameter of between 0.1 and 100 nm and preferably between 3 and 30 nm, and chosen, for example, from: silica, silica-alumina composites, cerium oxide, zirconium oxide, alumina, calcium carbonate, barium sulphate, calcium sulphate, zinc oxide and titanium dioxide. As silica-alumina composite colloidal particles that may be used in the compositions according to the invention, examples that may be mentioned include those sold by the company Grace under the names Ludox AM, Ludox AM-X 6021, Ludox HSA and Ludox TMA, and
(e) synthetic polymers, such as polyurethane latices or acrylic-silicone latices, in particular those described in patent application EP-1 038 519, such as a polydimethylsiloxane grafted with propylthio(polymethyl acrylate), propylthio(polymethyl methacrylate) and propylthio(polymethacrylic acid), or alternatively a polydimethylsiloxane grafted with propylthio(polyisobutyl methacrylate) and propylthio(polymethacrylic acid). Such grafted silicone polymers are especially sold by the company 3M under the trade names VS 80, VS 70 and LO21.

The tensioning agent will be present in the composition in an amount that is effective for obtaining the desired biological effect according to the invention. By way of example, the tensioning agent may be included in the composition according to the invention in a content ranging from 0.01% to 30% by weight of active material and preferably from 1% to 30% by weight of active material relative to the total weight of the composition.

The term "active material" is intended to exclude the medium in which the tensioning agent may be dissolved or dispersed in its commercial form, for example in the case of dispersions of colloidal particles.

It is also possible, especially for complementing and/or potentiating the effect of tensioning agents, to use agents for increasing the expression of mechanoreceptors, such as agents for increasing the expression of the integrins.
An example that may be mentioned is an extract of rye seed, such as the product sold by Silab under the name Coheliss^{®}.

### 16. Liporestructuring

According to the invention, the term "liporestructuring agents" means agents capable of stimulating lipogenesis and of promoting adipocyte differentiation, thus making it possible to prevent or slow down the loss of fat contained in the skin support tissues, which is also referred to as "loss of skin lipostructure".
The term "skin lipostructure" means the network of fat cells that forms the volumes on which facial skin sits and is moulded.

These agents are intended to reduce the loss of cutaneous density and/or the loss of skin lipostructure, in particular on the cheeks and in the area around the eyes, and/or prevent the collapse and/or hollowing of the volumes of the face, the loss of consistency of the skin and/or its support, in particular on the cheeks and in the area around the eyes, and/or improve the volumes underlying the skin of the face and/or the neck, in particular on the cheeks, the oval of the face and the area around the eyes, and/or improve the density, elasticity and support of the skin, in particular on the cheeks, the oval of the face and the area around the eyes, and/or remodel the facial features, in particular the oval of the face.

Examples of liporestructuring agents that may especially be mentioned include an extract of black tea, such as the extract of fermented black tea sold by Sederma under the name Kombuchka^{®}, and an extract of *Artemisia abrotanum*, such as the product sold by Silab under the name Pulpactyl^{®}.

### 17. Slimming agents

Slimming (lipolytic) agents that may especially be mentioned include caffeine, theophylline and its derivatives, theobromine, sericosine, Asiatic acid, acefylline, aminophylline, chloroethyltheophylline, diprofylline, diniprophylline, etamiphylline and its derivatives, etofylline and proxyphylline; extracts of tea, of coffee, of guarana, of maté, of cola (*Cola nitida*) and especially the dry extract of guarana fruit (*Paulina sorbilis*) containing 8% to 10% caffeine; extracts of climbing ivy (*Hedera helix*), of arnica (*Arnica montana L*), of rosemary (*Rosmarinus officinalis N*), of marigold (*Calendula officinalis*), of sage (*Salvia officinalis L*), of ginseng (*Panax ginseng*), of St. John's wort (*Hypericum perforatum*), of butcher's-broom (*Ruscus aculeatus L*), of meadowsweet (*Filipendula ulmaria L*), of orthosiphon (*Orthosiphon stamincus Benth*), of birch (*Betula alba*), of pumpwood and of argan tree, extracts of ginkgo biloba, extracts of horsetail, extracts of escin, extracts of cangzhu, extracts of *Chrysanthellum indicum*, extracts of diosgenin-rich Dioscorea plants or pure diosgenin or hecogenin and derivatives thereof, extracts of Ballota, extracts of *Guioa*, of *Davallia*, of *Terminalia*, of *Barringtonia*, of *Trema* or of *Antirobia*, the extract of bitter orange pips; an extract of husks of cocoa beans (*Theobroma cacao*) such as the product sold by Solabia under the name Caobromine^{®}.

### 18. Agents for promoting the cutaneous capillary circulation

The active agent acting on the cutaneous capillary circulation may be used for preventing dulling of the complexion and/or improving the appearance of the area around the eyes, particularly for reducing dark rings. It may be chosen, for example, from an extract of maritime pine bark, for instance Pycnogenol^{®} from Biolandes, manganese gluconate (Givobio GMn^{®} from SEPPIC), an extract of *Ammi visnaga* such as Visnadine from Indena, extract of lupin (Eclaline^{®} from Silab), the protein coupling of hydrolysed wheat/palmitic acid with palmitic acid, such as Epaline 100 from Laboratoires Carilène, the extract of bitter orange blossom (Remoduline^{®} from Silab), vitamin P and derivatives thereof, for instance methyl-4 esculetol sodium monoethanoate sold under the name Permethol^{®} by the company Sephytal, extracts of Ruscus, of common horse chestnut, of ivy, of ginseng and of melilot, caffeine, nicotinate and derivatives thereof, lysine and derivatives thereof, for instance Asparlyne^{®} from Solabia, an extract of black tea such as Kombuchka from Sederma; rutin salts; an extract of the alga *Corallina officinalis*, such as the product sold by Codif; and mixtures thereof.

As preferred agents for promoting the cutaneous capillary circulation, mention will be made of caffeine, an extract of bitter orange blossom, an extract of black tea, rutin salts and an extract of the alga *Corallina officinalis.*

### 19. Calmatives or anti-irritants

The term "calmative" means a compound that can reduce the sensation of stinging, itching or tautness of the skin.

As calmatives that may be used in the composition according to the invention, mention may be made of: procyanidol oligomers, vitamins E, C, B5 and B3, caffeine and derivatives thereof, pentacylic triterpenes and plant extracts containing them, β-glycyrrhetinic acid and salts or derivatives thereof (stearyl glycyrrhetate, 3-stearoyloxyglycyrrhetic acid or glycyrrhetinic acid monoglucuronide) and also plants containing them (e.g.: *Glycyrrhiza glabra*), oleanolic acid and salts thereof, ursolic acid and salts thereof, boswellic acid and salts thereof, betulinic acid and salts thereof, an extract of *Paeonia suffruticosa* and/or *lactiflora*, an extract of *Laminaria saccharina*, extracts of *Centella asiatica*, Canola oil, bisabolol, the phosphoric diester of vitamin E and C, for instance Sepivital EPC^{®} from SEPPIC, camomile extracts, allantoin, omega-3 unsaturated oils such as musk rose oil, blackcurrant oil, Ecchium oil, fish oil or beauty-leaf oil, plankton extracts, capryloyl glycine, a mixture of water lily blossom extract and of palmitoylproline, such as the product sold under the name Seppicalm VG^{®} by the company SEPPIC, an extract of *Boswellia serrata*, an extract of *Centipeda cunninghami*, such as the product sold under the name Cehami PF^{®} by the company TRI-K Industries, an extract of sunflower seeds, in particular Helioxine^{®} from Silab, an extract of *Linum usitatissimum* seeds, for instance Sensiline^{®} from Silab, tocotrienols, piperonal, an extract of *Epilobium angustifolium*, such as the product sold under the name Canadian Willowherb Extract by the company Fytokem Products, *Aloe vera*, phytosterols, cornflower water, rose water, an extract of mint, in particular of mint leaves, for instance Calmiskin^{®} from Silab, aniseed derivatives, filamentous bacteria, for instance *Vitreoscilla filiformis* as described in patent EP 761 204 sold by Chimex under the name Mexoryl SBG^{®}, an extract of rose petals, for instance Rose Flower Herbasol^{®} extract from the company Cosmetochem, shea butter, a mixture of the waxy fraction of barley seeds obtained by supercritical CO₂, of shea butter and of argan oil, for instance Stimu-tex AS^{®} from Pentapharm, alkaline-earth metal salts, especially strontium, a fermented extract of Alteromonas sold under the name Abyssine^{®} by the company Atrium Biotechnologies; spring water from the Vichy basin, such as waters originating from the Célestins, Chomel, Grande-Grille, Hôpital, Lucas and Parc sources, and preferably water from the Lucas source; an extract of *Eperua falcata* bark, such as the product sold by the company Cognis under the name Eperuline^{®}; an extract of *Paeonia suffruticosa* root, such as the product sold by the company Ichimaru Pharcos under the name Botanpi Liquid B^{®}; and mixtures thereof.

As preferred calmatives according to the invention, use will be made of:
β-glycyrrhetinic acid and salts or derivatives thereof (stearyl glycyrrhetate, 3-stearoyloxyglycyrrhetic acid or glycyrrhetinic acid monoglucuronide) and also plants containing them (e.g. *Glycyrrhiza glabra*); ursolic acid and salts thereof; extracts of *Centella asiatica*, Canola oil, bisabolol; camomile extracts, allantoin; a mixture of extract of water lily blossom and of palmitoylproline, such as the product sold under the name Seppicalm VG^{®} by the company SEPPIC; *Aloe vera*, rose water, extract of mint, in particular of mint leaves, such as Calmiskin^{®} from Silab, filamentous bacteria such as *Vitreoscilla filiformis* as described in patent EP 761 204 and sold by Chimex under the name Mexoryl SBG^{®}, an extract of rose petals such as Rose Flower Herbasol^{®} extract from the company Cosmetochem, shea butter, a fermented extract of Alteromonas sold under the name Abyssine^{®} by the company Atrium Biotechnologies; spring water from the Vichy basin, such as waters originating from the Célestins, Chomel, Grande-Grille, Hôpital, Lucas and Parc sources, and preferably water from the Lucas source; an extract of *Eperua falcata* bark, such as the product sold by the company Cognis under the name Eperuline^{®}; an extract of *Paeonia suffruticosa* root, such as the product sold by the company Ichimaru Pharcos under the name Botanpi Liquid B^{®}; and mixtures thereof.

### 20. Seboregulating or anti-seborrhoeic agents

The term "seboregulating or anti-seborrhoeic agents" especially means agents capable of regulating the activity of the sebaceous glands.

Mention may be made especially of:
- retinoic acid, benzoyl peroxide, sulphur, vitamin B6 (or pyridoxine), selenium chloride and sea fennel;
- mixtures of extract of cinnamon, of tea and of octanoylglycine such as Sepicontrol A5 TEA^{®} from SEPPIC;
- the mixture of cinnamon, sarcosine and octanoylglycine sold especially by the company SEPPIC under the trade name Sepicontrol A5^{®};
- zinc salts such as zinc gluconate, zinc pyrrolidonecarboxylate (or zinc pidolate), zinc lactate, zinc aspartate, zinc carboxylate, zinc salicylate and zinc cysteate;
- copper derivatives and in particular copper pidolate such as Cuivridone^{®} from Solabia;
- extracts of plants of the species *Arnica montana, Cinchona succirubra, Eugenia caryophyllata, Humulus lupulus*, *Hypericum perforatum*, *Mentha piperita*, *Rosmarinus officinalis*, *Salvia officinalis and Thymus vulgaris*, all sold, for example, by the company Maruzen;
- extracts of meadowsweet (*Spiraea ulmaria*), such as the product sold under the name Sebonormine^{®} by the company Silab;
- extracts of the alga *Laminaria saccharina*, such as the product sold under the name Phlorogine^{®} by the company Biotechmarine;
- mixtures of extracts of salad burnet root (*Sanguisorba officinalis*/*Poterium officinale*), of ginger rhizomes (*Zingiber officinalis*) and of cinnamon bark (*Cinnamomum cassia*), such as the product sold under the name Sebustop^{®} by the company Solabia;
- linseed extracts, such as the product sold under the name Linumine^{®} by the company Lucas Meyer;
- Phellodendron extracts, such as those sold under the name Phellodendron extract BG by the company Maruzen or Oubaku liquid B by the company Ichimaru Pharcos;
- mixtures of argan oil, of *Serenoa serrulata* (saw palmetto) extract and of sesame seed extract, such as the product sold under the name Regu SEB^{®} by the company Pentapharm;
- mixtures of extracts of willowherb, of *Terminalia chebula*, of nasturtium and of bioavailable zinc (microalgae), such as the product sold under the name Seborilys^{®} by the company Green Tech;
- extracts of *Pygeum afrianum*, such as the product sold under the name Pygeum afrianum sterolic lipid extract by the company Euromed;
- extracts of *Serenoa serrulata*, such as the products sold under the name Viapure Sabal by the company Actives International or those sold by the company Euromed;
- mixtures of extracts of plantain, of *Berberis aquifolium* and of sodium salicylate, such as the product sold under the name Seboclear^{®} by the company Rahn;
- clove extract, such as the product sold under the name Clove extract powder by the company Maruzen;
- argan oil, such as the product sold under the name Lipofructyl^{®} by Laboratoires Sérobiologiques;
- lactic protein filtrates, such as the product sold under the name Normaseb^{®} by the company Sederma;
- extracts of the alga *Laminaria*, such as the product sold under the name Laminarghane^{®} by the company Biotechmarine;
- oligosaccharides of the alga *Laminaria digitata*, such as the product sold under the name Phycosaccharide AC by the company Codif;
- sugar cane extracts, such as the product sold under the name Policosanol^{®} by the company Sabinsa;
- sulphonated shale oil, such as the product sold under the name Ichthyol Pale^{®} by the company Ichthyol;
- European meadowsweet *(Spiraea ulmaria)* extracts, such as the product sold under the name Cytobiol^{®} Ulmaire by the company Libiol;
- sebacic acid, especially sold in the form of a sodium polyacrylate gel under the name Sebosoft^{®} by the company Sederma;
- glucomannans extracted from konjac tuber and modified with alkylsulphonate chains, such as the product sold under the name Biopol Beta by the company Arch Chemical;
- extracts of *Sophora angustifolia*, such as those sold under the name Sophora powder or Sophora extract by the company Bioland;
- extracts of *Cinchona succirubra* bark, such as the product sold under the name Red Bark HS by the company Alban Muller;
- extracts of *Quillaja saponaria*, such as the product sold under the name Panama wood HS by the company Alban Muller;
- glycine grafted onto an undecylenic chain, such as the product sold under the name Lipacide UG OR by the company SEPPIC;
- the mixture of oleanolic acid and of nordihydroguaiaretic acid, such as the product sold in the form of a gel under the name AC.Net by the company Sederma;
- phthalimidoperoxyhexanoic acid;
- tri (C₁₂-C₁₃) alkyl citrate sold under the name Cosmacol^{®} ECI by the company Sasol; tri (C₁₄-C₁₅) alkyl citrate sold under the name Cosmacol^{®} ECL by the company Sasol;
- 10-hydroxydecanoic acid, and especially mixtures of 10-hydroxydecanoic acid, of sebacic acid and of 1,10-decanediol, such as the product sold under the name Acnacidol^{®} BG by the company Vincience; and
- mixtures thereof.

Preferred-seborrhoeic active agents that may be mentioned include:
- benzoyl peroxide and vitamin B6 (or pyridoxine),
- zinc salts such as zinc gluconate, zinc pyrrolidonecarboxylate (or zinc pidolate), zinc lactate, zinc aspartate, zinc carboxylate, zinc salicylate and zinc cysteate;
- meadowsweet *(Spiraea ulmaria)* extracts such as the product sold under the name Sebonormine^{®} by the company Silab;
- extracts of the alga *Laminaria saccharina*, such as the product sold under the name Phlorogine^{®} by the company Biotechmarine;
- mixtures of extracts of salad burnet root (*Sanguisorba officinalis*/*Poterium officinale*), of ginger rhizomes (*Zingiber officinalis*) and of cinnamon bark (*Cinnamomum cassia*), such as the product sold under the name Sebustop^{®} by the company Solabia;
- clove extract, such as the product sold under the name Clove extract powder by the company Maruzen;
- lactic protein filtrates, such as the product sold under the name Normaseb^{®} by the company Sederma;
- European meadowsweet (*Spiraea ulmaria*) extracts, such as the product sold under the name Cytobiol^{®} Ulmaire by the company Libiol;
- sebacic acid, especially sold in the form of a sodium polyacrylate gel under the name Sebosoft^{®} by the company Sederma;
- glycine grafted onto an undecylenic chain, such as the product sold under the name Lipacide UG OR by the company SEPPIC;
- tri (C₁₂-C₁₃) alkyl citrate sold under the name Cosmacol^{®} ECI by the company Sasol; tri (C₁₄-C₁₅) alkyl citrate sold under the name Cosmacol^{®} ECL by the company Sasol;
- 10-hydroxydecanoic acid, and especially mixtures of 10-hydroxydecanoic acid, of sebacic acid and of 1,10-decanediol, such as the product sold under the name Acnacidol^{®} BG by the company Vincience;
- and mixtures thereof.

Preferentially, the anti-seborrhoeic active agent is chosen from:
- zinc salts such as zinc gluconate, zinc pyrrolidonecarboxylate (or zinc pidolate), zinc lactate, zinc aspartate, zinc carboxylate, zinc salicylate and zinc cysteate; and preferably zinc pyrrolidonecarboxylate (or zinc pidolate) or zinc salicylate;
- clove extract, such as the product sold under the name Clove extract powder by the company Maruzen;
- glycine grafted onto an undecylenic chain, such as the product sold under the name Lipacide UG OR by the company SEPPIC;
- tri (C₁₂-C₁₃) alkyl citrate sold under the name Cosmacol^{®} ECI by the company Sasol; tri (C₁₄-C₁₅) alkyl citrate sold under the name Cosmacol^{®} ECL by the company Sasol;
- and mixtures thereof.

The anti-seborrhoeic active agent is, for example, present in a content ranging from 0.1% to 10% by weight, preferably from 0.1% to 5% by weight, and preferentially from 0.5% to 3% by weight, relative to the total weight of the composition.

### 21. Astringents

According to the invention, the term "astringents" means agents for combatting the dilation of the sebaceous follicles.

As astringents that may be used in the composition according to the invention, mention may be made of extracts of mushroom pulp (*Polyporus officinalis*), for instance "Laricyl LS8865^{®}" from Cognis, extracts of *Terminalia catappa* and *Sambucus nigra,* for instance Phytofirm LS9120^{®} from Cognis, extracts of gall nut, for instance Tanlex VE^{®} from Ichimaru Pharcos, aluminium hydroxychloride, centella extracts (e.g. Plantactiv centella from Cognis), dicetyl dimethyl ammonium chloride, for instance Varisoft 432 CG^{®} from Degussa, common horse chestnut extracts, mallow extracts, witch hazel extracts, sweet almond extracts, marshmallow root extracts and linseed extracts, for instance Almondermin LS 3380^{®} from Cognis, burdock extracts, nettle extracts, birch extracts, horsetail extracts, camomile extracts, for instance those sold under the name Extrapone 9 special^{®} by the company Symrise, skullcap extracts, European meadowsweet extracts (for example Cytobiol Ulmaire from Libiol), a mixture of extracts of white ginger, of horsetail, of nettle, of rosemary and of yucca, for instance Herb extract B1348^{®} from Bell flavors & fragrances, extracts of acacia, of elm, of white willow, of cinnamon, of birch and of meadowsweet, panama sapogenins, zinc phenolsulphonate from Interchemical, extracts of gentian, of cucumber and of walnut, the mixture of extracts of Ratanhia, of grapefruit, of gumweed and of oak gall, for instance Epilami^{®} from Alban Muller.

As preferred astringents according to the invention, use will be made of skullcap extracts, European meadowsweet extracts, meadowsweet extracts, gentian extracts and burdock extracts, and mixtures thereof.

### 22. Cicatrizing agents

Examples of cicatrizing agents that may especially be mentioned include:
allantoin, urea, certain amino acids, for instance hydroxyproline, arginine, and serine, and also extracts of white lily (for instance Phytélène Lys 37EG 16295 from Indena), a yeast extract, for instance the cicatrizing agent LS LO/7225B from Laboratoires Serobiologiques), tamanu oil, extract of *Saccharomyces cerevisiae*, for instance Biodynes^{®} TRF^{®} from Arch Chemical, oat extracts, chitosan and derivatives, for instance chitosan glutamate, carrot extracts, artemia extract, for instance GP4G^{®} from Vincience, sodium acexamate, lavandin extracts, propolis extracts, ximenynic acid and salts thereof, rosehip oil, marigold extracts, for instance Souci Ami^{®} Liposolible from Alban Muller, horsetail extracts, lemon peel extracts, for instance Herbasol^{®} citron from Cosmetochem, helichrysum extracts, common yarrow extracts and folic acid.

As preferred cicatrizing agents according to the invention, use will be made of arginine, serine, folic acid, tamanu oil, sodium acexamate, horsetail extracts and helichrysum extracts, and mixtures thereof.

### 23. Anti-inflammatory agents

As particular anti-inflammatory agents that may be used according to the invention, mention may be made of cortisone, hydrocortisone, indomethacin, betamethasone, azelaic acid, acetaminophen, diclofenac, clobetasol propionate, folic acid; an extract of *Eperua falcata* bark, such as the product sold by the company Cognis under the name Eperuline^{®}; an extract of *Paeonia suffruticosa* root, such as the product sold by the company Ichimaru Pharcos under the name Botanpi Liquid B^{®}; and mixtures thereof.

Preferred anti-inflammatory agents that will be mentioned are azelaic acid, folic acid, an extract of *Eperua falcata* bark, such as the product sold by the company Cognis under the name Eperuline^{®}; an extract of *Paeonia suffruticosa* root, such as the product sold by the company Ichimaru Pharcos under the name Botanpi Liquid B^{®}; and mixtures thereof.

### 24. Anti-acne agents

In one advantageous aspect of the invention, the composition may also comprise at least one anti-acne active agent.

The term "anti-acne active agent" especially means any active agent that has effects on the specific flora of greasy skin, for instance *Propionibacterium acnes* (*P. acnes*). These effects may be bactericidal.

Antibactericidal active agents that may especially be mentioned include:
- active agents and preserving agents with antimicrobial activity mentioned in patent application DE 103 24 567, which is incorporated into the present invention by reference,
- Asiatic acid,
- the monoethanolamine salt of 1-hydroxy-4-methyl 6-trimethylpentyl-2-pyridone (INCI name: piroctone olamine), sold especially under the name Octopirox^{®} by the company Clariant;
- citronellic acid, perillic acid (or 4-isopropenylcyclohex-1-enecarboxylic acid),
- glyceryl 2-ethylhexyl ether (INCI name: ethylhexylglycerine), for example sold under the name Sensiva SC 50^{®} by the company Shulke & Mayr,
- glyceryl caprylate/caprate, for example sold under the name Capmul MCM^{®} by the company Abitec;
- sodium calcium phosphosilicate, especially sold under the names Bioactive Glasspowder^{®} and Actysse Premier BG^{®} by the company Schott Glass;
- silver-based particles, for example those sold under the name Metashine ME 2025 PS^{®} by the company Nippon Sheet Glass;
- hop cone extract (*Humulus lupulus*) obtained by supercritical CO₂ extraction, such as the product sold under the name HOP CO₂-TO extract^{®} by the company Flavex Naturextrakte,
- St. John's Wort extract obtained by supercritical CO₂ extraction, such as the product sold under the name St. John's Wort CO₂-TO extract^{®} by the company Flavex Naturextrakte,
- a mixture of extracts of roots of *Scutellaria baicalensis,* of *Paeonia suffruticosa* and of *Glycyrrhiza glabra*, such as the product sold under the name BMB - CF^{®} by the company Naturogin,
- argan tree extract, for instance Argapure LS9710^{®} from Cognis;
- bearberry leaf extracts, for instance the product sold under the name Melfade-J by the company Pentapharm;
- 10-hydroxy-2-decanoic acid such as Acnacidol P^{®} from Vincience, sodium ursolate, azelaic acid, diiodomethyl p-tolyl sulphone such as Amical Flowable^{®} from Angus, malachite powder, zinc oxide such as Zincare^{®} from Elementis GmbH, octadecenedioic acid such as Arlatone dioic DCA^{®} from Uniqema; ellagic acid; 2,4,4'-trichloro-2'-hydroxydiphenyl ether (or triclosan), 1-(3',4'-dichlorophenyl)-3-(4'-chlorophenyl)urea (or triclocarban), 3,4,4'-trichlorocarbanilide, 3',4',5'-trichlorosalicylanilide, phenoxyethanol, phenoxypropanol, phenoxyisopropanol, hexamidine isethionate, metronidazole and salts thereof, miconazole and salts thereof, itraconazole, terconazole, econazole, ketoconazole, saperconazole, fluconazole, clotrimazole, butoconazole, oxiconazole, sulfaconazole, sulconazole, terbinafine, ciclopirox, ciclopiroxolamine, undecylenic acid and salts thereof, benzoyl peroxide, 3-hydroxybenzoic acid, 4-hydroxybenzoic acid, phytic acid, N-acetyl-L-cysteine, lipoic acid, azelaic acid and salts thereof, arachidonic acid, resorcinol, 3,4,4'-trichlorocarbanalide, octoxyglycerine or octoglycerine, octanoylglycine such as Lipacid C8G^{®} from SEPPIC, caprylyl glycol, 10-hydroxy-2-decanoic acid, dichlorophenylimidazoledioxolane and derivatives thereof described in patent application WO 93/18743, iodopropynyl butylcarbamate, 3,7,11-trimethyldodeca-2,5,10-trienol or farnesol, phytosphingosines; quaternary ammonium salts, for instance cetyltrimethylammonium salts and cetylpyridinium salts, and
- mixtures thereof.

Mention may also be made of certain surfactants with an antimicrobial effect, for instance sodium cocoamphoacetate or disodium diacetate such as Miranol C2M Conc. NP, betaines, for instance the cocoyl betaine Genagen KB from Clariant, sodium lauryl ether sulphate, for instance Emal 270 D from Kao, decyl glucoside, for instance Plantacare 2000 UP, branched C₁₂-₁₃ dialkyl malates, for instance Cosmacol EMI, propylene glycol monoesters, for instance propylene glycol monolaurate, monocaprylate or monocaprate, lauryldimethylamine betaine, for instance Empigen BB/LS, and also polyquaternary ammoniums such as Quaternium-24 or Bardac 2050 from Lonza and those described in patent FR 0 108 283, and mixtures thereof.

As preferred antimicrobial agents, an agent chosen from octoglycerine or octoxyglycerine, and 10-hydroxy-2-decanoic acid, and mixtures thereof, will be used in the compositions of the invention.

Other additional anti-acne active agents may be added to the abovementioned anti-acne active agents.

Mention may be made especially of active agents with bacterial anti-adhesion effects or agents that act on the biofilm of bacteria to prevent them from multiplying.

As agents for preventing and/or reducing the adhesion of microorganisms, mention may be made especially of: phytanetriol and derivatives thereof as described in patent application EP 1 529 523, plant oils such as wheatgerm oil, calendula oil, castor oil, olive oil, avocado oil, sweet almond oil, groundnut oil, jojoba oil, sesame oil, apricot kernel oil, sunflower oil and macadamia oil, described in patent EP 1 133 979, or certain surfactants such as disodium cocoamphodiacetate, oxyethylenated (7 EO) glyceryl cocoate, 18-hexadecenyl succinate, octoxyglyceryl palmitate, octoxyglyceryl behenate, dioctyl adipate, PPG-15 stearyl ether, and the branched C₁₂-C₁₃ dialkyl tartrates described in patent EP 1 129 694, and mixtures thereof.
In particular with regard to the propagation of P. *acnes*, or as active agents that act on the biofilm of bacteria to prevent them from proliferating, mention may be made of pentylene glycol, Nylon-66 (polyamide 66 fibres), rice bran oil, polyvinyl alcohol such as Celvol 540 PV alcohol^{®} from Celanese Chemical, rapeseed oil such as Akorex L^{®} from Karlshamns, and fructose derivatives, and mixtures thereof.

The anti-acne active agent may be present in a content ranging from 0.01% to 10% by weight and preferably from 0.05% to 5% by weight relative to the total weight of the composition.

As a function of the nature and/or solubility of the abovementioned active agents, a person skilled in the art will know how to select the most suitable embodiment according to the invention.
As lipophilic active agents that may be used in the kit or at least one of the compositions of the invention, mention may be made especially of D-α-tocopherol, DL-α-tocopherol, D-α-tocopheryl acetate, DL-α-tocopheryl acetate, ascorbyl palmitate, vitamin F glycerides, D vitamins, vitamin D2, vitamin D3, retinol, retinol esters, retinyl palmitate, retinyl propionate, carotenes including β-carotene, D-panthenol, farnesol, farnesyl acetate, salicylic acid and derivatives thereof, for instance 5-n-octanoylsalicylic acid, α-hydroxy acid alkyl esters such as citric acid, lactic acid, glycolic acid, Asiatic acid, madecassic acid, asiaticoside, the total extract of *Centella asiatica*, β-glycyrrhetinic acid, α-bisabolol, ceramides, for instance 2-oleoylamino-1,3-octadecane, phytanetriol, phospholipids of marine origin rich in polyunsaturated essential fatty acids, ethoxyquine, rosemary extract, balm extract, quercetin, extract of dried microalgae, essential oil of bergamot, octyl methoxycinnamate, butylmethoxydibenzoylmethane, octyl triazone, 3,5-di-tert-butyl-4-hydroxy-3-benzylidenecamphor, antibiotics, antifungal agents, anaesthetics, analgesics, antiseptics, antiviral agents, pesticides and herbicides, and mixtures thereof.

The cosmetic and/or dermatological active agents will be present in the kit or one of the compositions according to the invention in a content ranging from 0.001% to 20% by weight relative to the total weight of the composition, preferably from 0.01% to 10%, even more preferentially from 0.5% to 5% and more preferably from 0.1% to 1% by weight relative to the total weight of the composition.

For "scrubbing" applications, the contents of cosmetic and/or dermatological active agents may range from 1% to 50% by weight relative to the total weight of the composition and preferably from 1% to 30% by weight relative to the total weight of the composition.

Scrubbing is a well-known means for improving the appearance and/or texture of the skin and/or the scalp, especially for improving the radiance and homogeneity of the complexion and/or for reducing the visible and/or tactile irregularities of the skin, and in particular for improving the surface appearance of the skin, for attenuating actinic lentigo, acne or chicken pox marks, and also for preventing, attenuating or combatting the signs of aging of the skin, and especially for smoothing out irregularities in the texture of the skin, such as wrinkles and fine lines.

It has the effect of removing a surface part of the skin to be treated (epidermis and possibly the upper layer of the dermis), via chemical methods.

### OTHER ADDITIONAL INGREDIENTS

To complement and/or optimize the effects imparted by the cosmetic and/or dermatological active agents mentioned above on the keratin materials, it may be advantageous to incorporate into the compositions of the invention other additional ingredients.

In particular, these additional ingredients may impart an immediate visual effect that will be relayed by the biological effect of the active agents mentioned above. They may also, via a mechanical action (e.g.: abrasive fillers), amplify the effect of the biological active agents mentioned above.

Thus, the composition according to the invention may also comprise at least one agent chosen from matting agents, fillers with a soft-focus effect, fluorescers, agents for promoting the naturally pinkish coloration of the skin, abrasive fillers or exfoliants, and mixtures thereof.

### Matting agents

The term "matting agent" means agents intended to make the skin visibly more matt and less shiny.

The matting effect of the agent and/or composition containing it may especially be evaluated using a gonioreflectometer, by measuring the ratio R between the specular reflection and the scattered reflection. A value of R of less than or equal to 2 generally reflects a matting effect.

The matting agent may especially be chosen from a rice starch or a corn starch, kaolinite, talc, a pumpkin seed extract, cellulose microbeads, plant fibres, synthetic fibres, in particular polyamide fibres, expanded acrylic copolymer microspheres, polyamide powders, silica powders, polytetrafluoroethylene powders, silicone resin powders, acrylic polymer powders, wax powders, polyethylene powders, powders of elastomeric crosslinked organopolysiloxane coated with silicone resin, talc/titanium dioxide/alumina/silica composite powders, amorphous mixed silicate powders, silicate particles and especially mixed silicate particles, and mixtures thereof.

Examples of matting agents that may especially be mentioned include:
- rice or corn starch, in particular an aluminium starch octenyl succinate sold under the name Dry Flo^{®} by the company National Starch;
- kaolinite;
- silicas;
- talc;
- a pumpkin seed extract as sold under the name Curbilene^{®} by the company Indena;
- cellulose microbeads as described in patent application EP 1 562 562;
- fibres, such as silk fibre, cotton fibre, wool fibre, flax fibre, cellulose fibre extracted especially from wood, from vegetables or from algae, polyamide fibre (Nylon^{®}), modified cellulose fibre, poly-p-phenyleneterephthamide fibre, acrylic fibre, polyolefin fibre, glass fibre, silica fibre, aramid fibre, carbon fibre, Teflon^{®} fibre, insoluble collagen fibre, polyester fibre, polyvinyl chloride or polyvinylidene chloride fibre, polyvinyl alcohol fibre, polyacrylonitrile fibre, chitosan fibre, polyurethane fibre, polyethylene phthalate fibre, fibres formed from a mixture of polymers, resorbable synthetic fibres, and mixtures thereof described in patent application EP 1 151 742;
- expanded acrylic copolymer microspheres such as those sold by the company Expancel under the name Expancel 551^{®};
- fillers with an optical effect as described in patent application FR 2 869 796, in particular:
- polyamide powders (Nylon^{®}), for instance Nylon 12 particles of the Orgasol type from Arkema, with a mean size of 10 microns and a refractive index of 1.54,
- silica powders, for instance Silica beads SB150 from Miyoshi with a mean size of 5 microns and a refractive index of 1.45,
- polytetrafluoroethylene powders, for instance PTFE Ceridust 9205F from Clariant, with a mean size of 8 microns and a refractive index of 1.36,
- silicone resin powders, for instance the silicone resin Tospearl 145A from GE Silicone with a mean size of 4.5 microns and a refractive index of 1.41,
- acrylic copolymer powders, especially of polymethyl(meth)acrylate, for instance the PMMA particles Jurymer MBI from Nihon Junyoki, with a mean size of 8 microns and a refractive index of 1.49, or the Micropearl M100^{®} and F 80 ED^{®} particles from the company Matsumoto Yushi-Seiyaku,
- wax powders, for instance the paraffin wax particles Microease 114S from Micropowders, with a mean size of 7 microns and a refractive index of 1.54,
- polyethylene powders, especially comprising at least one ethylene/acrylic acid copolymer, and in particular constituted of ethylene/acrylic acid copolymers, for instance the particles Flobeads EA 209 from Sumitomo (with a mean size of 10 microns and a refractive index of 1.48),
- elastomeric crosslinked organopolysiloxane powders coated with silicone resin, especially with silsesquioxane resin, as described, for example, in patent US 5 538 793. Such elastomeric powders are sold under the names KSP-100, KSP-101, KSP-102, KSP-103, KSP-104 and KSP-105 by the company Shin-Etsu, and
- talc/titanium dioxide/alumina/silica composite powders such as those sold under the name Coverleaf^{®} AR-80 by the company Catalyst & Chemicals,
- mixtures thereof,
- compounds that absorb and/or adsorb sebum as described in patent application FR 2 869 796. Mention may be made especially of:
- silica powders, for instance the porous silica microspheres sold under the name Silica Beads SB-700 sold by the company Miyoshi, the products Sunsphere^{®} H51, Sunsphere^{®} H33 and Sunsphere^{®} H53 sold by the company Asahi Glass; the polydimethylsiloxane-coated amorphous silica microspheres sold under the names SA Sunsphere^{®} H-33 and SA Sunsphere^{®} H-53 sold by the company Asahi Glass;
- amorphous mixed silicate powders, especially of aluminium and magnesium, for instance the product sold under the name Neusilin UFL2 by the company Sumitomo;
- polyamide (Nylon^{®}) powders, for instance Orgasol^{®} 4000 sold by the company Arkema, and
- acrylic polymer powders, especially of polymethyl methacrylate, for instance Covabead^{®} LH85 sold by the company Wacker; of polymethyl methacrylate/ethylene glycol dimethacrylate, for instance Dow Corning 5640 Microsponge^{®} Skin Oil Adsorber sold by the company Dow Corning, or Ganzpearl^{®} GMP-0820 sold by the company Ganz Chemical; of polyallyl methacrylate/ethylene glycol dimethacrylate, for instance Poly-Pore^{®} L200 or Poly-Pore^{®} E200 sold by the company Amcol; of ethylene glycol dimethacrylate/lauryl methacrylate copolymer, for instance Polytrap^{®} 6603 sold by the company Dow Corning;
- silicate particles, such as alumina silicate;
- mixed silicate particles, such as:
- magnesium aluminium silicate particles, such as saponite or hydrated magnesium aluminium silicate with a sodium sulphate sold under the trade name Sumecton^{®} by the company Kunimine;
- the magnesium silicate, hydroxyethylcellulose, black cumin oil, marrow oil and phospholipids complex or Matipure^{®} from Lucas Meyer, and
- mixtures thereof.

Preferred matting agents that may be used according to the invention include a pumpkin seed extract, a rice or corn starch, kaolinite, silicas, talc, polyamide powders, polyethylene powders, acrylic copolymer powders, expanded acrylic copolymer microspheres, silicone resin microbeads and mixed silicate particles, and mixtures thereof.

### Fillers with a soft-focus effect

These fillers may be any material capable of modifying and hiding wrinkles by virtue of their intrinsic physical properties. These fillers may especially modify wrinkles via a tensioning effect, a covering effect or a soft-focus effect.

Examples of fillers that may be given include the following compounds:
- porous silica microparticles, for instance the Silica Beads^{®} SB150 and SB700 from Miyoshi with a mean size of 5 µm; the series H Sunspheres^{®} from Asahi Glass, for instance Sunspheres H33 and H51 with respective sizes of 3.5 and 5 µm;
- hollow hemispherical silicone resin particles such as NLK 500^{®}, NLK 506^{®} and NLK 510^{®} from Takemoto Oil and Fat, especially described in EP-A-1 579 849;
- silicone resin powders, for instance the silicone resin Tospearl^{®} 145A from GE Silicone, with a mean size of 4.5 µm;
- acrylic copolymer powders, especially of polymethyl (meth)acrylate, for instance the PMMA particles Jurymer MBI^{®} from Nihon Junyoki, with a mean size of 8 µm, the hollow PMMA spheres sold under the name Covabead^{®} LH85 by the company Wacker, and vinylidene/acrylonitrile/ methylene methacrylate expanded microspheres sold under the name Expancel^{®};
- wax powders, for instance the paraffin wax particles MicroEase^{®} 114S from MicroPowders, with a mean size of 7 µm;
- polyethylene powders, especially comprising at least one ethylene/acrylic acid copolymer, for instance the Flobeads^{®} EA 209 E from Sumimoto, with a mean size of 10 µm;
- crosslinked elastomeric organopolysiloxane powders coated with silicone resin and especially with silsesquioxane resin, sold under the names KSP-100^{®}, KSP-101^{®}, KSP-102^{®}, KSP 103^{®}, KSP-104^{®} and KSP-105^{®} by the company Shin-Etsu;
- talc/titanium dioxide/alumina/silica composite powders, for instance those sold under the name Coverleaf AR-80^{®} by the company Catalyst & Chemical;
- talc, mica, kaolin, lauryl glycine, starch powders crosslinked with octenyl succinate anhydride, boron nitride, polytetrafluoroethylene powders, precipitated calcium carbonate, magnesium carbonate, magnesium hydrogen carbonate, barium sulphate, hydroxyapatite, calcium silicate, cerium dioxide and glass or ceramic microcapsules;
- hydrophilic or hydrophobic, synthetic or natural, mineral or organic fibres such as silk fibres, cotton fibres, wool fibres, flax fibres, cellulose fibres extracted especially from wood, vegetables or algae, polyamide (Nylon^{®}) fibres, modified cellulose fibres, poly-p-phenylene terephthamide fibres, acrylic fibres, polyolefin fibres, glass fibres, silica fibres, aramid fibres, carbon fibres, polytetrafluoroethylene (Teflon^{®}) fibres, insoluble collagen fibres, polyester fibres, polyvinyl chloride fibres, polyvinylidene chloride fibres, polyvinyl alcohol fibres, polyacrylonitrile fibres, chitosan fibres, polyurethane fibres, polyethylene phthalate fibres, fibres formed from a mixture of polymers, resorbable synthetic fibres, and mixtures thereof described in patent application EP 1 151 742;
- spherical elastomeric crosslinked silicones, for instance Trefil E-505C^{®} or E-506C^{®} from Dow Corning;
- abrasive fillers, which, via a mechanical effect, smooth out the skin microrelief, such as abrasive silica, for instance Abrasif SP^{®} from Semanez or nut or shell powders (for example of apricot or walnut, from Cosmetochem).

The fillers with an effect on the signs of aging are especially chosen from porous silica microparticles, hollow hemispherical silicone particles, silicone resin powders, acrylic copolymer powders, polyethylene powders, crosslinked elastomeric organopolysiloxanes powders coated with silicone resin, talc/titanium dioxide/alumina/silica composite powders, precipitated calcium carbonate, magnesium carbonate, magnesium hydrogen carbonate, barium sulphate, hydroxyapatite, calcium silicate, cerium dioxide, glass or ceramic microcapsules, and silk fibres or cotton fibres, and mixtures thereof.

The filler may be a soft-focus filler.
The term "soft-focus" filler means a filler which in addition gives the complexion transparency and a hazy effect. Preferably, the soft-focus fillers have a mean particle size of less than or equal to 15 microns. These particles may be in any form and in particular may be spherical or non-spherical. These fillers are more preferably non-spherical.
The soft-focus fillers may be chosen from silica and silicate powders, especially alumina powder, powders of polymethyl methacrylate (PMMA) type, talc, silica/TiO₂ or silica/zinc oxide composites, polyethylene powders, starch powders, polyamide powders, styrene/acrylic copolymer powders and silicone elastomers, and mixtures thereof.
Mention may be made in particular of talc with a number-average size of less than or equal to 3 microns, for example talc with a number-average size of 1.8 microns and especially the product sold under the trade name Talc P3^{®} by the company Nippon Talc, Nylon^{®} 12 powder, especially the product sold under the name Orgasol 2002 Extra D Nat Cos^{®} by the company Atochem, silica particles 1% to 2% surface-treated with a mineral wax (INCI name: hydrated silica (and) paraffin) such as the products sold by the company Degussa, amorphous silica microspheres, such as the products sold under the name Sunsphere, for example of reference H-53^{®} by the company Asahi Glass, and silica microbeads such as those sold under the name SB-700^{®} or SB-150^{®} by the company Miyoshi, this list not being limiting.
The concentration of these fillers with an effect on the signs of aging in the compositions according to the invention may be between 0.1% and 40%, or even between 0.1% and 20% by weight, relative to the total weight of the composition.

### Fluorescers

The term "fluorescer" means a substance which, under the effect of ultraviolet rays and/or visible light, re-emits in the visible region the portion of light that it has absorbed under the same colour as that which it naturally reflects. The naturally reflected colour is thus reinforced by the re-emitted colour and appears extremely bright.

Examples that may be mentioned include coloured polyamide and/or formaldehyde/benzoguanamine and/or melamine/formaldehyde/sulphonamide resins, from coloured aminotriazine/formaldehyde/sulphonamide co-condensates and/or from metallized polyester flakes and/or mixtures thereof. These fluorescent pigments may also be present in the form of aqueous dispersions of fluorescent pigments.

Mention may also be made of the pink-coloured fluorescent aminotriazine/formaldehyde/sulphonamide co-condensate with a mean particle size of 3-4 microns sold under the trade name "Fiesta Astral Pink FEX-1" and the blue-coloured fluorescent aminotriazine/ formaldehyde/sulphonamide co-condensate with a mean particle size of 3-4.5 microns sold under the trade name "Fiesta Comet Blue FTX-60" by the company Swada, or alternatively the yellow-coloured benzoguanamine/ formaldehyde resin covered with formaldehyde/urea resin sold under the trade name "FB-205 Yellow" and the red-coloured benzoguanamine/formaldehyde resin covered with formaldehyde/urea resin sold under the trade name "FB-400 Orange Red" by the company UK Seung Chemical, and the orange-coloured polyamide resin sold under the trade name "Flare 911 Orange 4" by the company Sterling Industrial Colors.

The fluorescent substances are preferably present in the composition in a content ranging from 0.1% to 20%, preferably from 0.1% to 15% and more preferably from 0.5% to 3% by weight relative to the total weight of the composition.
When the organic fluorescent substances are white, they are also known as optical brighteners.

The optical brightener has the effect of intensifying the radiance and reviving the shades of cosmetic compositions comprising them on application to the skin.
Among the optical brighteners that may be mentioned more particularly are stilbene derivatives, in particular polystyrylstilbenes and triazinestilbenes, coumarin derivatives, in particular hydroxycoumarins and aminocoumarins, oxazole, benzoxazole, imidazole, triazole and pyrazoline derivatives, pyrene derivatives and porphyrin derivatives, and/or mixtures thereof.

Such compounds are available, for example, under the trade names Tinopal SOP^{®} and Uvitex OB^{®} from the company Ciba Geigy.

The optical brighteners preferentially used are sodium 4,4'-bis[(4,6-dianilino-1,3,5-triazin-2-yl)amino]stilbene-2,2'-disulphonate, 2,5-thiophenediylbis(5-tert-butyl-1,3-benzoxazole) and disodium 4,4'-distyrylbiphenylsulphonate, and/or mixtures thereof.

### Agents for promoting the naturally pinkish coloration of the skin

Mention may be made especially of:
- a self-tanning agent, i.e. an agent which, when applied to the skin, especially to the face, can produce a tan effect that is more or less similar in appearance to that which may result from prolonged exposure to the sun (natural tan) or under a UV lamp;
- an additional colouring agent, i.e. any compound that has particular affinity for the skin, which allows it to give the skin a lasting, non-covering coloration (i.e. that does not have a tendency to opacify the skin) and that is not removed either with water or using a solvent, and that withstands both rubbing and washing with a solution containing surfactants. Such a lasting coloration is thus distinguished from the superficial and transient coloration provided, for example, by a makeup pigment;
and mixtures thereof.

Examples of self-tanning agents that may especially be mentioned include:
- dihydroxyacetone (DHA),
- erythrulose, and
- the combination of a catalytic system formed from:
- manganese and/or zinc oxide salts, and
- alkali metal and/or alkaline-earth metal hydrogen carbonates.

The self-tanning agents are generally chosen from monocarbonyl or polycarbonyl compounds, for instance isatin, alloxan, ninhydrin, glyceraldehyde, mesotartaric aldehyde, glutaraldehyde, erythrulose, pyrazoline-4,5-dione derivatives as described in patent application FR 2 466 492 and WO 97/35842, dihydroxyacetone (DHA) and 4,4-dihydroxypyrazolin-5-one derivatives as described in patent application EP 903 342. DHA will preferably be used.

The DHA may be used in free and/or encapsulated form, for example in lipid vesicles such as liposomes, especially described in patent application WO 97/25970.

In general, the self-tanning agent is present in an amount ranging from 0.01% to 20% by weight and preferably in an amount of between 0.1% and 10% of the total weight of the composition.

Other dyes that allow modification of the colour produced by the self-tanning agent may also be used.

These dyes may be chosen from synthetic or natural direct dyes.

These dyes may be chosen, for example, from red or orange dyes of the fluorane type such as those described in patent application FR 2 840 806. Mention may be made, for example, of the following dyes:
- tetrabromofluoresceine or eosin known under the CTFA name: CI 45380 or Red 21;
- phloxin B known under the CTFA name: CI 45410 or Red 27;
- diiodofluoresceine known under the CTFA name: CI 45425 or Orange 10;
- dibromofluoresceine known under the CTFA name: CI 45370 or Orange 5;
- the sodium salt of tetrabromofluoresceine known under the CTFA name: CI 45380 (Na salt) or Red 22;
- the sodium salt of phloxin B known under the CTFA name: CI 45410 (Na salt) or Red 28;
- the sodium salt of diiodofluoresceine known under the CTFA name: CI 45425 (Na salt) or Orange 11;
- erythrosine known under the CTFA name: CI 45430 or Acid Red 51;
- phloxin known under the CTFA name: CI 45405 or Acid Red 98.

These dyes may also be chosen from anthraquinones, caramel, carmine, carbon black, azulene blues, methoxalene, trioxalene, guajazulene, chamuzulene, Bengal rose, cosine 10B, cyanosin and daphinin.

These dyes may also be chosen from indole derivatives, for instance the monohydroxyindoles as described in patent FR 2 651 126 (i.e.: 4-, 5-, 6- or 7-hydroxyindole) or the dihydroxyindoles as described in patent EP-B-0 425 324 (i.e.: 5,6-dihydroxyindole, 2-methyl-5,6-dihydroxyindole, 3-methyl-5,6-dihydroxyindole or 2,3-dimethyl-5,6-dihydroxyindole).

### Abrasive fillers or exfoliants

As exfoliants that may be used in rinse-out compositions according to the invention, examples that may be mentioned include exfoliant or scrubbing particles of mineral, plant or organic origin. Thus, polyethylene beads or powder, Nylon powder, polyvinyl chloride powder, pumice powder, ground apricot kernel or walnut husk, sawdust, glass beads and alumina, and mixtures thereof, may be used, for example.

Mention may also be made of Exfogreen^{®} from Solabia (bamboo extract), extracts of strawberry akenes (Strawberry Akenes from Greentech), peach kernel powder, apricot kernel powder, and finally, in the field of plant powders with an abrasive effect, mention may be made of cranberry kernel powder.

As abrasive fillers or exfoliants that are preferred according to the invention, mention will be made of peach kernel powder, apricot kernel powder, cranberry kernel powder, strawberry akene extracts and bamboo extracts.

The examples which follow serve to illustrate the invention without, however, being limiting in nature. In these examples, the amounts of the ingredients of the compositions are given as % by weight relative to the total weight of the composition.

**EXAMPLES 1 to 4**

| Phase | Ingredients | Ex 1 (*) | Ex 2 (*) | Ex 3 (*) | Ex 4 |
|---|---|---|---|---|---|
| A | Glycerol | 4 | 4 | 4 | 4 |
| | Propylene glycol | 4 | 4 | 4 | 4 |
| | Sequestering agent | 0.1 | 0.1 | 0.1 | 0.1 |
| | Water | qs 100 | qs 100 | qs 100 | qs 100 |
| B | Ethoxylated sodium ethyldiamido-n-cocoyl sulphonate (15EO)/behenyl alcohol/ stearate/glyceryl citrate mixture (ceralution H) | 2 | 2 | 2 | 2 |
| | Cetyl alcohol | 0.5 | 0.5 | 0.5 | 0.5 |
| | Isononyl isononanoate | 4 | 4 | 4 | 4 |
| | 2-ethylhexyl 2-cyano-3,3-diphenylacrylate | 10 | 10 | 10 | 10 |
| | 4-tert-butyl-4'-methoxydibenzoylmethane | 3 | 3 | 3 | 3 |
| | 2-ethylhexyl salicylate | 5 | 5 | 5 | 5 |
| | Preserving agent | 0.25 | 0.25 | 0.25 | 0.25 |
| | Preserving agent | 1 | 1 | 1 | 1 |
| C | Mixture of natural tocopherols in soybean oil (50/50) | 0.2 | 0.2 | 0.2 | 0.2 |
| | Fragrance | 0.4 | 0.4 | 0.4 | 0.4 |
| E | Triethanolamine | | 0.2 | 0.16 | 0.5 |
| D | Methacrylic acid/ethyl acrylate/oxyethylenated (25EO) behenyl methacrylate terpolymer as an aqueous emulsion (Aculyn 28) (associated polymer) | | 1 | | |
| | Xanthan gum | | | 0.1 | |
| | Crosslinked methacrylic acid/ethyl acrylate copolymer in an emulsion at 33% with respect to active material (Carbopol Aqua SF1) | | | | 1.67 |

| | | | | | |
|---|---|---|---|---|---|
| (*) not part of the invention | | | | | |

### Protocol of examples 1 to 4:

- Phase A is prepared and then heated to 65°C. Phase B is prepared and then B is heated to 65°C. The emulsion is prepared by pouring B into A, with vigorous stirring for 15 min. D is introduced into the emulsion with gentle stirring (scraper blade), followed by neutralization with (E). C is introduced into the emulsion with gentle stirring (scraper blade). The emulsion is left to cool to ambient temperature with gentle stirring.

### Stability and viscosity tests

The viscosity of each formulation at time T=0 and after two months is then measured. The stability of each emulsion after two months of 45°C is also observed.

The results obtained are summarized:

| Composition | **Ex 1 (*)** | **Ex 2 (*)** | **Ex 3 (*)** | **Ex 4** |
|---|---|---|---|---|
| Viscosity at T = 0 (rotor 2) | 17 | 240 mPa.s | 180 mPa.s | 182 mPa.s |
| Viscosity at T = 2 months (rotor 2) | nd | 245 mPa.s | nd | 170 mPa.s |
| Stability 2 months 45°C | Unstable 3 weeks | Stable | Unstable 24 hours | Stable |

| | | | | |
|---|---|---|---|---|
| nd = not determinable | | | | |

It is noted that only example 4 according to the invention, comprising a gemini surfactant and a crosslinked copolymer of methacrylic acid/ethyl acrylate, remains very fluid (viscosity less than 200 mPa.s) and stable over time (2 months), unlike examples 1 and 3.

In example 2, the presence of an associative polymer results in an emulsion that is less fluid (240-245 mPa.s).

## Claims

1. Photoprotective composition containing, in a cosmetically acceptable medium,
a) as liquid phase, an oil-in-water emulsion, emulsified with at least one dimeric surfactant comprising two surfactant units, which may be identical or different, each being constituted of a hydrophilic head and a hydrophobic tail and connected to one another, via the hydrophilic heads, by means of a spacer group, said dimeric surfactant being chosen from compounds of formula (I): 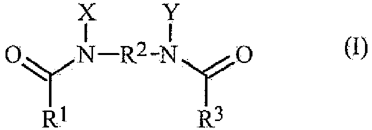 in which
R¹ and R³ represent a linear or branched C₅-C₂₅ alkyl group, which is saturated or contains up to two non-vicinal unsaturations,
R² represents a C₁-C₁₂ alkylene group,
X and Y each represent a (C₂H₄O)x(C₃H₆O)y-RF group with x = 0 - 15,
y = 0-10, x + y ≥ 1, and RF = -SO₃M, -CH₂-CO₂M, -P(O) (OM)₂, H, -C₃H₆SO₃M, or a -CH₂(CHOH)₄CH₂OH group when x + y = 0, and
M represents an alkali metal ion, (alkyl)ammonium, alkanolammonium, H or a 1/2 alkaline-earth metal ion,
b) at least one organic UV screen and/or at least one mineral UV screen, and
c) at least one crosslinked copolymer of methacrylic acid and **ethyl**acrylate.

2. Composition according to Claim 1, in which the dimeric surfactant is chosen from the compounds of formula (I): 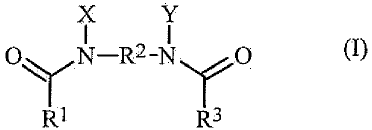 in which
R¹ and R³ are identical and represent a C₈-C₁₆ alkyl group,
R² represents a C₂-C₈ alkylene group,
X and Y each represent a -(C₂H₄O)x-RF group with x = 10-15 and RF = -SO₃M, in which M is an alkali metal atom.

3. Composition according to Claim 1 or 2, in which the dimeric surfactant of formula (I) is sodium dicocoylethylenediamine PEG-15 sulphate of formula:

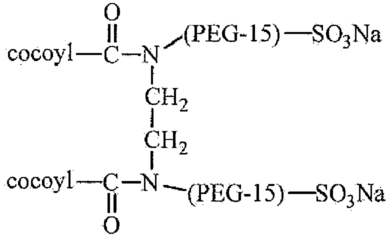

4. Composition according to any one of Claims 1 to 3, **characterized in that** it comprises one of the following mixtures:
a) behenyl alcohol, glyceryl stearate, glyceryl stearate citrate and sodium dicocoylethylenediamine PEG-15 sulphate;
b) sodium lauroyl lactylate and sodium dicocoylethylenediamine PEG-15 sulphate;
c) aqua, capric/caprylic triglyceride, glycerin, ceteareth-25, sodium dicocoylethylenediamine PEG-15 sulphate, sodium lauroyl lactylate, behenyl alcohol, glyceryl stearate, glyceryl stearate citrate, gum arabic, xanthan gum, phenoxyethanol, methylparaben, ethylparaben, butylparaben and isobutylparaben.

5. Composition according to any one of Claims 1 to 4, **characterized in that** it comprises the mixture behenyl alcohol, glyceryl stearate, glyceryl stearate citrate and sodium dicocoylethylenediamine Peg-15 sulphate.

6. Composition according to any one of Claims 1 to 5, **characterized in that**, in the crosslinked copolymer of methacrylic acid and of **ethyl**acrylate,
(i) the methacrylic acid is present in amounts ranging from 20% to 80% by weight, and more particularly from 25% to 70% by weight, and even more particularly from 35% to 65% by weight, relative to the total weight of the copolymer,
(ii) the ethyl acrylate is present in amounts ranging from 15% to 80% by weight, and more particularly from 25% to 75% by weight, and even more particularly from 35% to 65% by weight, relative to the total weight of the copolymer.

7. Composition according to any one of Claims 1 to 6, **characterized in that** the copolymer of methacrylic acid and of **ethyl**acrylate is crosslinked with at least one ethylenically polyunsaturated crosslinking agent.

8. Composition according to any one of Claims 1 to 7, **characterized in that** the crosslinked copolymer of methacrylic acid and of **ethyl**acrylate is in the form of a dispersion in water.

9. Composition according to Claim 8, **characterized in that** the average size of the particles of the copolymer in the dispersion is generally between 10 and 500 nm, and preferably between 20 and 200 nm, and more preferentially from 50 to 150 nm.

10. Composition according to any one of Claims 1 to 9, in which the organic screens are chosen from anthranilates; dibenzoylmethane derivatives; cinnamic derivatives; salicylic derivatives, camphor derivatives; benzophenone derivatives; β,β-diphenylacrylate derivatives; triazine derivatives; benzotriazole derivatives; benzalmalonate derivatives; benzimidazole derivatives; imidazolines; bisbenzoazolyl derivatives; p-aminobenzoic acid (PABA) derivatives; methylenebis(hydroxyphenyl-benzotriazole) derivatives; benzoxazole derivatives; screening polymers and screening silicones; α-alkylstyrene-derived dimers; 4,4-diarylbutadienes; merocyanin derivatives, and mixtures thereof.

11. Composition according to any one of the preceding claims, **characterized in that** the inorganic UV screen(s) is (are) chosen from coated or uncoated metal oxide pigments having an average primary particle size of: between 5 nm and 100 nm, preferably between 10 nm and 50 nm.

12. Composition according to Claim 11, **characterized in that** the metal oxide-based pigments are pigments of titanium oxide, of iron oxide, of zinc oxide, of zirconium oxide or of cerium oxide, which have optionally been coated.

## Patentansprüche

1. Lichtschutzzusammensetzung, die in einem kosmetisch unbedenklichen Medium
a) als flüssige Phase eine Öl-in-Wasser-Emulsion, die mit mindestens einem dimeren Tensid mit zwei Tensid-Einheiten, die gleich oder verschieden sind und jeweils aus einem hydrophilen Kopf und einem hydrophoben Schwanz bestehen und mit Hilfe einer Spacer-Gruppe über die hydrophoben Köpfe miteinander verbunden sind, emulgiert ist,
wobei das dimere Tensid aus Verbindungen der Formel (I): 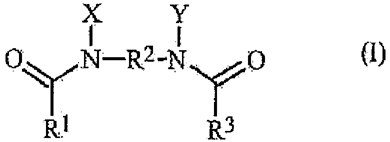 ausgewählt ist, wobei
R¹ und R³ für eine lineare oder verzweigte C₅-C₂₅-Alkylgruppe, die gesättigt ist oder bis zu zwei nicht vicinale Ungesättigtheiten enthält, stehen, R² für eine C₁-C₁₂-Alkylengruppe steht,
X und Y jeweils für eine (C₂H₄O)x(C₃H₆O)y-RF-Gruppe mit x = 0 - 15 stehen,
y = 0-10, x + y ≥ 1 und RF = -SO₃M, -CH₂-CO₂M, -P(O)(OM)₂, H, -C₃H₆SO₃M oder eine -CH₂(CHOH)₄CH₂OH-Gruppe, wenn x + y = 0, und
M für ein Alkalimetallion, (Alkyl)ammonium, Alkanolammonium, H oder ein 1/2 Erdalkalimetallion steht,
b) mindestens einen organischen UV-Filter und/oder mindestens einen mineralischen UV-Filter und
c) mindestens ein vernetztes Copolymer von Methacrylsäure und Ethylacrylat
enthält.

2. Zusammensetzung nach Anspruch 1, wobei das dimere Tensid aus den Verbindungen der Formel (I): 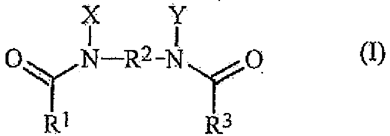 ausgewählt ist, wobei
R¹ und R³ gleich sind und für eine C₈-C₁₆-Alkylgruppe stehen,
R² für eine C₂-C₈-Alkylengruppe steht,
X und Y jeweils für eine -(C₂H₄O)x-RF-Gruppe mit x = 10 - 15 und RF = -SO₃M stehen, wobei M für ein Alkalimetallatom steht.

3. Zusammensetzung nach Anspruch 1 oder 2, wobei es sich bei dem dimeren Tensid der Formel (I) um Natriumdicocoylethylendiamin-PEG-15-sulfat der Formel: 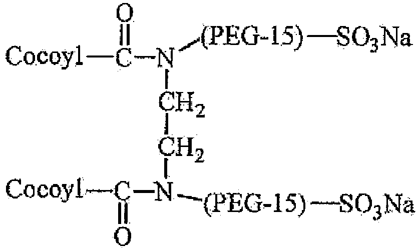 handelt.

4. Zusammensetzung nach einem der Ansprüche 1 bis 3, **dadurch gekennzeichnet, dass** sie eine der folgenden Mischungen umfasst:
a) Behenylalkohol, Glycerylstearat, Glyceryl-stearatcitrat und Natriumdicocoylethylendiamin-PEG-15-sulfat;
b) Natriumlauroyllactylat und Natriumdicocoylethylendiamin-PEG-15-sulfat;
c) Aqua, Caprin/Capryltriglycerid, Glycerin, Ceteareth-25, Natriumdicocoylethylendiamin-PEG-15-sulfat, Natriumlauroyllactylat, Behenyl-alkohol, Glycerylstearat, Glycerylstearat-citrat, Gummi arabicum, Xanthangummi, Phenoxyethanol, Methylparaben, Ethylparaben, Butyl-paraben und Isobutylparaben.

5. Zusammensetzung nach einem der Ansprüche 1 bis 4, **dadurch gekennzeichnet, dass** sie die Mischung Behenylalkohol, Glycerylstearat, Glycerylstearat-citrat und Natriumdicocoylethylendiamin-PEG-15-sulfat umfasst.

6. Zusammensetzung nach einem der Ansprüche 1 bis 5, **dadurch gekennzeichnet, dass** in dem vernetzten Copolymer von Methacrylsäure und Ethylacrylat
(i) die Methacrylsäure in Mengen im Bereich von 20 bis 80 Gew.-% und spezieller von 25 bis 70 Gew.-% und noch spezieller von 35 bis 65 Gew.-%, bezogen auf das Gesamtgewicht des Copolymers, vorliegt,
(ii) das Ethylacrylat in Mengen im Bereich von 15 bis 80 Gew.-% und spezieller von 25 bis 75 Gew.-% und noch spezieller von 35 bis 65 Gew.-%, bezogen auf das Gesamtgewicht des Copolymers, vorliegt.

7. Zusammensetzung nach einem der Ansprüche 1 bis 6, **dadurch gekennzeichnet, dass** das Copolymer von Methacrylsäure und Ethylacrylat mit mindestens einem mehrfach ethylenisch ungesättigten Vernetzungsmittel vernetzt ist.

8. Zusammensetzung nach einem der Ansprüche 1 bis 7, **dadurch gekennzeichnet, dass** das vernetzte Copolymer von Methacrylsäure und Ethylacrylat in Form einer Dispersion in Wasser vorlegt.

9. Zusammensetzung nach Anspruch 8, **dadurch gekennzeichnet, dass** die mittlere Größe der Teilchen des Copolymers in der Dispersion im Allgemeinen zwischen 10 und 500 nm und vorzugsweise zwischen 20 und 200 nm liegt und weiter bevorzugt 50 bis 150 nm beträgt.

10. Zusammensetzung nach einem der Ansprüche 1 bis 9, wobei die organischen Filter aus Anthranilaten; Dibenzoylmethanderivaten; Zimtsäurederivaten; Salicylsäurederivaten, Campherderivaten; Benzophenonderivaten; β,β-Diphenylacrylatderivaten; Triazinderivaten; Benzotriazolderivaten; Benzalmalonatderivaten; Benzimidazolderivaten; Imidazolinen; Bisbenzoazolylderivaten; p-Aminobenzoesäurederivaten (PABA-Derivaten); Methylenbis-(hydroxyphenylbenzotriazol)derivaten; Benzoxazol-derivaten; Filterpolymeren und Filtersilikonen; von α-Alkylstyrol abgeleiteten Dimeren; 4,4-Diarylbutadienen; Merocyaninderivaten und Mischungen davon ausgewählt sind.

11. Zusammensetzung nach einem der vorhergehenden Ansprüche, **dadurch gekennzeichnet, dass** der anorganische UV-Filter bzw. die anorganischen UV-Filter aus beschichteten oder unbeschichteten Metalloxidpigmenten mit einer mittleren Primärteilchengröße zwischen 5 nm und 100 nm, vorzugsweise zwischen 10 nm 50 nm, ausgewählt ist bzw. sind.

12. Zusammensetzung nach Anspruch 11, **dadurch gekennzeichnet, dass** es sich bei den auf Metalloxid basierenden Pigmenten um gegebenenfalls beschichtete Pigmente aus Titanoxid, Eisenoxid, Zinkoxid, Zirconiumoxid oder Ceroxid handelt.

## Revendications

1. Composition photoprotectrice contenant, dans un milieu cosmétiquement acceptable,
a) en tant que phase liquide, une émulsion huile-dans-eau, émulsionnée par au moins un agent tensioactif dimère comportant deux motifs tensioactifs, identiques ou différents, constitués chacun d'une tête hydrophile et d'une queue hydrophobe et reliés l'un à l'autre, au niveau des têtes hydrophiles, par un groupe espaceur,
ledit agent tensioactif dimère étant choisi parmi des composés de formule (I) : 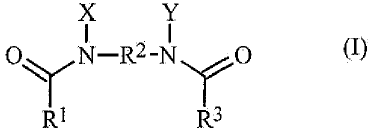 où
R¹ et R³ représentent un groupe alkyle en C₅-C₂₅, linéaire ou ramifié, saturé ou contenant jusqu'à deux insaturations non voisines,
R² représente un groupe alkylène en C₁-C₁₂,
X et Y représentent chacun un groupe (C₂H₄O)x(C₃H₆O)y-RF avec x = 0 - 15,
y = 0-10, x + y ≥ 1, et RF = -SO₃M, -CH₂-CO₂M, -P(O)(OM)₂, H, -C₃H₆SO₃M, ou un groupe -CH₂(CHOH)₄CH₂OH lorsque x + y = 0, et
M représente un ion alcalin, (alkyl)ammonium, alcanolammonium, H ou un 1/2 ion alcalinoterreux,
b) au moins un filtre UV organique e/ou au moins un filtre UV minéral, et
c) au moins un copolymère réticulé d'acide méthacrylique et d'acrylate d'éthyle

2. Composition selon la revendication 1, où le tensioactif dimère est choisi parmi les composés de formule (I) : 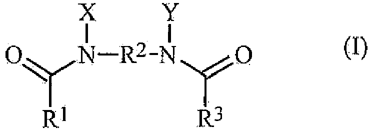 où
R¹ et R³ sont identiques et représentent un groupe alkyle en n C₈-C₁₆,
R² représente un groupe alkylène en C₂-C₈,
X et Y représentent chacun un groupe -(C₂H₄O)x-RF avec x = 10-15 et RF = -SO₃M, où M est un atome de métal alcalin.

3. Composition selon la revendication 1 ou 2, où le tensioactif dimère de formule (I) est le Sodium Dicocoylethylenediamine PEG-15 Sulfate de formule :

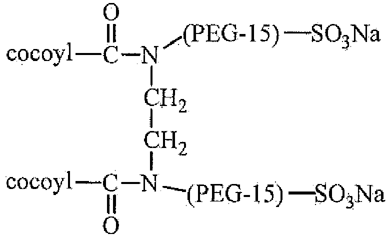

4. Composition selon l'une quelconque des revendications 1 à 3, **caractérisée par le fait qu'**elle comprend l'un des mélanges suivants :
a) Behenyl Alcohol, Glyceryl Stearate, Glyceryl Stearate Citrate et Sodium Dicocoylethylenediamine PEG-15 Sulfate ;
b) Sodium Lauroyl Lactylate et Sodium Dicocoylethylènediamine PEG-15 Sulfate ;
c) Aqua, Capric/Caprylic Triglyceride, Glycerin, Ceteareth-25, Sodium Dicocoylethylenediamine PEG-15 Sulfate, Sodium Lauroyl Lactylate, Behenyl Alcohol, Glyceryl Stearate, Glyceryl Stearate Citrate, Gum Arabic, Xanthan Gum, Phenoxyethanol, Methylparaben, Ethylparaben, Butylparaben, Isobutylparaben ;

5. Composition selon l'une quelconque des revendications 1 à 4, **caractérisée par le fait qu'**elle comprend le mélange Behenyl Alcohol, Glyceryl Stearate, Glyceryl Stearate Citrate et Sodium Dicocoylethylenediamine PEG-15 Sulfate.

6. Composition selon l'une quelconque des revendications 1 à 5, **caractérisée par le fait que** dans le copolymère réticulé d'acide méthacrylique et d'acrylate d'éthyle,
(i) l'acide méthacrylique est présent dans des quantités allant de 20 à 80% en poids et plus particulièrement de 25 à 70 % en poids et encore plus particulièrement de 35 à 65% en poids par rapport au poids total du copolymère,
(ii) l'acrylate d'éthyle est présent dans des quantités allant de 15 à 80% en poids et plus particulièrement de 25 à 75 % en poids et encore plus particulièrement de 35 à 65% en poids par rapport au poids total du copolymère.

7. Composition selon l'une quelconque des revendications 1 à 6, **caractérisée par le fait que** le copolymère d'acide méthacrylique et d'acrylate d'éthyle est réticulé par au moins un agent réticulant éthylèniquement polyinsaturé.

8. Composition selon l'une quelconque des revendications 1 à 7, **caractérisée par le fait que** le copolymère réticulé d'acide méthacrylique et d'acrylate d'éthyle se présente sous forme de dispersion dans l'eau.

9. Composition selon la revendication 8, **caractérisée par le fait que** la taille moyenne des particules du copolymère dans la dispersion est généralement comprise entre 10 et 500 nm et de préférence entre 20 et 200 nm et plus préférentiellement de 50 à 150 nm.

10. Composition selon l'une quelconque des revendications 1 à 9, où les filtres organiques sont choisis parmi les anthranilates ; les dérivés de dibenzoylméthane ; les dérivés cinnamiques ; les dérivés salicyliques, les dérivés du camphre ; les dérivés de la benzophénone ; les dérivés de β, β-diphénylacrylate ; les dérivés de triazine ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes ; les dérivés de mérocyanines et leurs mélanges.

11. Composition selon l'une quelconque des revendications précédentes, **caractérisée par le fait que** le ou les filtres UV inorganiques sont choisis parmi les pigments d'oxydes métalliques enrobés ou non de taille moyenne de particules primaires comprise entre 5 nm et 100 nm, de préférence entre 10 nm et 50 nm.

12. Composition selon la revendication 11, **caractérisée par le fait que** les pigments à base d'oxyde métallique sont des pigments d'oxyde de titane, d'oxyde de fer, d'oxyde de zinc, d'oxyde de zirconium ou d'oxyde de cérium, éventuellement enrobés .
